# EUROPEAN PATENT APPLICATION

(11) **EP 3 933 853 A1**
(43) Date of publication of application: **05.01.2022**
(21) Application number: 21181414.0
(22) Date of filing: 24.06.2021
(51) Int. Cl.: G16H 80/00

(54) **METHOD FOR SUPPORTING EXPERT MEETING BY USING COMPUTER, SUPPORT APPARATUS, COMPUTER PROGRAM FOR SUPPORTING EXPERT MEETING, AND SUPPORT SYSTEM**

(30) Priority: 30.06.2020 JP 2020113102
(71) Applicant: SYSMEX CORPORATION, Kobe-shi Hyogo 651-0073 (JP)
(72) Inventor: Ikeda, Koji, Hyogo, 651-0073 (JP); Takahata, Takayuki, Hyogo, 651-0073 (JP); Onoe, Hiroko, Hyogo, 651-0073 (JP)
(74) Representative: Hoffmann Eitle

(57) **Abstract**

Disclosed is a support method for supporting, by using a computer, management of an expert meeting in which a plurality of medical workers interpret a result of a panel test for a patient, and the support method includes: obtaining request information of a plurality of panel tests for patients for each of whom the expert meeting is held; generating information of predicted meeting frames that are related to a number of meetings and/or a meeting time and that are predicted on a scheduled date for holding the expert meeting, based on the obtained request information of the plurality of panel tests; and generating divergence information indicating a difference between the predicted meeting frames and set meeting frames that are related to a number of meetings and/or a meeting time and that are preset on the scheduled date, such that the difference is recognizable.

## Description

### CROSS REFERENCE TO RELATED APPLICATIONS

This application claims priority from prior Japanese Patent Application No. 2020-113102, filed on June 30, 2020, entitled "METHOD FOR SUPPORTING EXPERT MEETING BY USING COMPUTER, SUPPORT APPARATUS, COMPUTER PROGRAM FOR SUPPORTING EXPERT MEETING, AND SUPPORT SYSTEM", the entire content of which is incorporated herein by reference.

### FIELD OF THE INVENTION

The present invention relates to a method for supporting expert meetings by using a computer, a support apparatus, a computer program for supporting expert meetings, and a support system.

### BACKGROUND

In recent years, research for cancer genomic medicine has been promoted in cancer treatment. In cancer genomic medicine, a gene panel test for performing comprehensive and simultaneous examination of a lot of gene mutations for each patient by using a next-generation sequencer, is performed to determine a treatment strategy that fits to the patient based on the results of the test.

Japanese Laid-Open Patent Publication No. 2018-533123 discloses an information platform for collecting electronic medical charts, pathologic images, and results of tests such as a gene panel test as distributed in some medical institution, and supporting determination of a treatment strategy for a patient.

In genomic medicine, the treatment strategy is determined via an expert meeting of which the members are a genetic counselor, a researcher of molecular genetics, a clinical laboratory technician, a bioinformatician, and the like in addition to a doctor in charge of the patient, and a pathologist. The expert meetings are often held for a plurality of cases on the same day, and the number of the meetings per one day is preset. The number of test requests are expected to be increased in the future. The number of the test requests is not expected to be always constant throughout a year, and the test requests may be concentrated at some times while the number of the test requests may be relatively small at other times. In such circumstances, scheduled dates for holding the expert meetings and the number of meetings per one day need to be optimized by, for example, adding a scheduled meeting date or extending a meeting time per one day as necessary in order to shorten a period from a test request to determination of a treatment strategy. However, Japanese Laid-Open Patent Publication No. 2018-533123 does not disclose a technique for optimizing scheduled dates for holding expert meetings and the number of the meetings per one day.

Therefore, an object of the present invention is to provide a method for supporting management of expert meetings by using a computer, a support apparatus, a computer program, and a support system for easily optimizing scheduled dates for holding expert meetings and the number of the meetings per one day.

### SUMMARY OF THE INVENTION

An embodiment of the present invention is directed to a support method for supporting, by using a computer, management of an expert meeting in which a plurality of medical workers interpret a result of a panel test for a patient. The support method includes: obtaining request information of a plurality of panel tests for patients for each of whom the expert meeting is held; generating information of predicted meeting frames that are related to a number of meetings and/or a meeting time and that are predicted on a scheduled date for holding the expert meeting, based on the obtained request information of the plurality of panel tests; and generating divergence information indicating a difference between the predicted meeting frames and set meeting frames that are related to a number of meetings and/or a meeting time and that are preset on the scheduled date, such that the difference is recognizable.

Another embodiment of the present invention is directed to a support apparatus (A) for supporting, by using a computer, management of an expert meeting in which a plurality of medical workers interpret a result of a panel test for a patient. The support apparatus (A) includes a controller (100A). The controller (100A) obtains request information of a plurality of panel tests for patients for each of whom the expert meeting is held, generates information of predicted meeting frames that are related to a number of meetings and/or a meeting time and that are predicted on a scheduled date for holding the expert meeting, based on the obtained request information of the plurality of panel tests; and generates divergence information indicating a difference between the predicted meeting frames and set meeting frames that are related to a number of meetings and/or a meeting time and that are preset on the scheduled date, such that the difference is recognizable.

Another embodiment of the present invention is directed to a computer program for supporting, by using a computer, management of an expert meeting in which a plurality of medical workers interpret gene information of a patient. The computer program causes the computer to perform the steps of: obtaining request information of a plurality of panel tests for patients for each of whom the expert meeting is held; generating information of predicted meeting frames that are related to a number of meetings and/or a meeting time and that are predicted on a scheduled date for holding the expert meeting, based on the obtained request information of the plurality of panel tests; and generating divergence information indicating a difference between the predicted meeting frames and set meeting frames that are related to a number of meetings and/or a meeting time and that are preset on the scheduled date, such that the difference is recognizable.

Another embodiment of the present invention is directed to a support system (1000) for supporting, by using a computer, management of an expert meeting in which a plurality of medical workers interpret gene information of a patient. The support system (1000) includes: a support apparatus (A) including a controller (100A); and one or a plurality of computers (B10, B20, G30). The controller (100A) of the support apparatus (A) obtains request information of a plurality of panel tests for patients for each of whom the expert meeting is held, generates information of predicted meeting frames that are related to a number of meetings and/or a meeting time and that are predicted on a scheduled date for holding the expert meeting, based on the obtained request information of the plurality of panel tests, and generates divergence information indicating a difference between the predicted meeting frames and set meeting frames that are related to a number of meetings and/or a meeting time and that are preset on the scheduled date, such that the difference is recognizable. The one or the plurality of computers (B10, B20, G30) obtain and display the divergence information.

A method for supporting management of expert meetings by using a computer, a support apparatus, a computer program, and a support system can be provided for easily optimizing scheduled dates for holding expert meetings and the number of the meetings per one day.

### BRIEF DESCRIPTION OF THE DRAWINGS

FIG. 1 shows a flow of genomic medicine;
FIG. 2 shows a flow of a gene panel test;
FIG. 3A shows an outline of a method for supporting management of expert meetings according to an embodiment;
FIG. 3B shows an example of a flow of setting a candidate date for an expert meeting in which a test result is deliberated, based on request information of a gene panel test.
FIG. 4 shows a hardware configuration of a system 1000;
FIG. 5 shows a hardware configuration of an integrated data management device A;
FIG. 6 shows a functional block of a controller of the integrated data management device A;
FIG. 7 schematically shows a master table M and an expert meeting schedule database SDB;
FIG. 8 shows a hardware configuration of a clinical information management device B10, B20, G30;
FIG. 9 shows a functional block of a controller of the clinical information management device B10, B20, G30;
FIG. 10 shows a hardware configuration of a test information management device C11;
FIG. 11 shows a functional block of a controller of the test information management device C11;
FIG. 12 shows a hardware configuration of an expert meeting terminal B15, B25, G35, C15, SP11, SP15;
FIG. 13 shows a functional block of a controller of the expert meeting terminal B15, B25, G35 in a medical institution;
FIG. 14 shows a functional block of each of a controller of the expert meeting terminal C15 in a test facility C1, and a controller of an expert meeting terminal SP11 in an external facility;
FIG. 15 shows a functional block of a controller of the bureau expert meeting terminal SP15;
FIG. 16 is a flow chart showing a part of an operation of the system 1000;
FIG. 17 is a flow chart showing a part of the operation of the system 1000;
FIG. 18 shows an example of a graphical user interface UIa;
FIG. 19 is a flow chart showing a part of the operation of the system 1000;
FIG. 20 is a flow chart showing a part of an operation of the integrated data management device A;
FIG. 21 is a flow chart showing a part of the operation of the integrated data management device A;
FIG. 22A shows an example of setting an allotment date;
FIG. 22B shows an example of setting an allotment date
FIG. 23 shows an example of setting the predicted number of the meetings;
FIG. 24 is a flow chart showing a part of the operation of the integrated data management device A;
FIG. 25 is a flow chart showing a part of the operation of the integrated data management device A;
FIG. 26 shows an example of display information;
FIG. 27 is a flow chart showing a part of the operation of the integrated data management device A;
FIG. 28 shows an example of a graphical user interface;
FIG. 29 shows an example of a dialogue;
FIG. 30 is a flow chart showing a part of the operation of the integrated data management device A;
FIG. 31A shows an example of a dialogue;
FIG. 31B shows an example of the updated expert meeting schedule database SDB;
FIG. 32 shows an example of a graphical user interface;
FIG. 33 shows an example of a graphical user interface;
FIG. 34 shows an example of a graphical user interface;
FIG. 35 shows an example of a graphical user interface;
FIG. 36 shows an example of a graphical user interface;
FIG. 37 shows an example of a graphical user interface;
FIG. 38 shows an example of a graphical user interface;
FIG. 39 is a flow chart showing a part of the operation of the integrated data management device A;
FIG. 40A shows an example of a reservation dialogue;
FIG. 40B shows an example of display information for FIG. 40A;
FIG. 40C shows an example of a reservation dialogue;
FIG. 40D shows an example of display information for FIG. 40C;
FIG. 41 is a flow chart showing a part of the operation of the integrated data management device A;
FIG. 42A shows genomic medicine according to another embodiment; and
FIG. 42B shows a correspondence between a required-time table T and the master table M.

### DETAILED DESCRIPTION OF THE PREFERRED EMBODIMENTS

Embodiments of the present invention will be described below in detail with reference to the accompanying drawings. In the following description and drawings, the same reference character denotes the same or similar component. Therefore, description for the same or similar components will be omitted.

### I. Outlines of flows of genomic medicine and gene panel test, and method for supporting management of expert meetings according to the present embodiment

Embodiments of the present invention are directed to a method for supporting management of expert meetings by using a computer, a support apparatus, a computer program, and a support system.

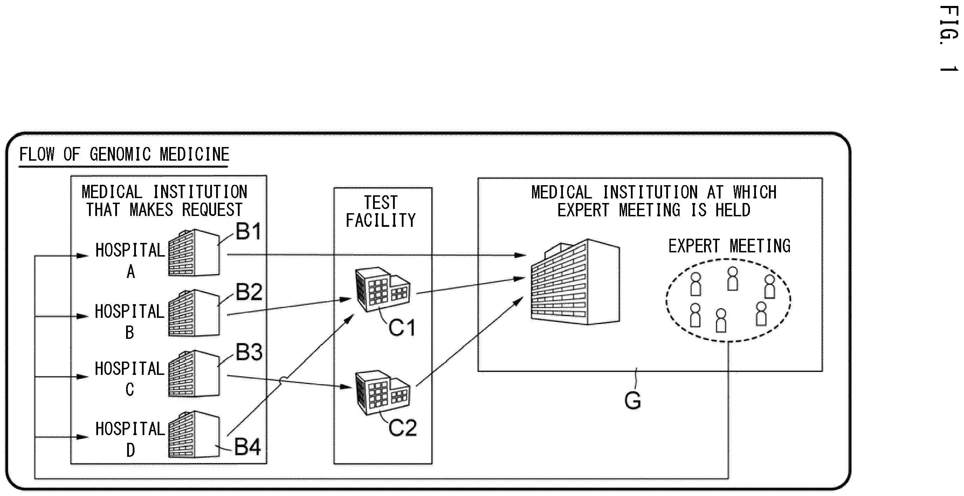

FIG. 1 shows a flow of genomic medicine. Genomic medicine is medicine in which analysis of a nucleic acid sequence of a disease-related gene is performed by using a specimen from a patient, and a treatment effective for the disease of the patient is determined to perform the treatment. The disease is, but is not particularly limited to, for example, cancer. The analysis of a nucleic acid sequence of a disease-related gene with use of a specimen from a patient is also called a gene panel test. In the description herein, the "panel" is intended to represent a set of genes to be analyzed. In the gene panel test, several tens to several hundreds of kinds of genes can be analyzed in one test. The result of the gene panel test is not interpreted only by a doctor in charge of the patient but interpreted in an expert meeting (also called expert panel) participated in by a group of experts including the doctor in charge, a pathologist who performs tissue diagnosis, a clinical laboratory technician and/or a bioinformatician who perform the gene panel test, a genetic counselor and/or a molecular genetics researcher who are experts of gene mutation interpretation, and/or the like. In the expert meeting, a treatment strategy that fits to the patient who has undergone the gene panel test is determined. As shown in FIG. 1, the expert meeting is usually held at a medical institution G for which the expert works. The experts who work for institutions other than the medical institution G may visit the medical institution G to participate in the expert meeting or may participate in the expert meeting by using on-line meeting systems at the institutions for which they work. The medical institution G may receive a request for holding the expert meeting from one or more of medical institutions B1, B2, B3, and B4. Such a medical institution G is called a hub hospital in Japan. A hospital that requests the hub hospital to hold the expert meeting is called an affiliated hospital in Japan. The hub hospital can also independently perform the gene panel test. The medical institutions B1, B2, B3, and B4 usually have no testing equipment for performing the gene panel test, and thus request a test facility C1, C2 or the hub hospital to perform the gene panel test. Feedback of the results from the expert meeting is made to the doctor in charge in the medical institution that made the request.

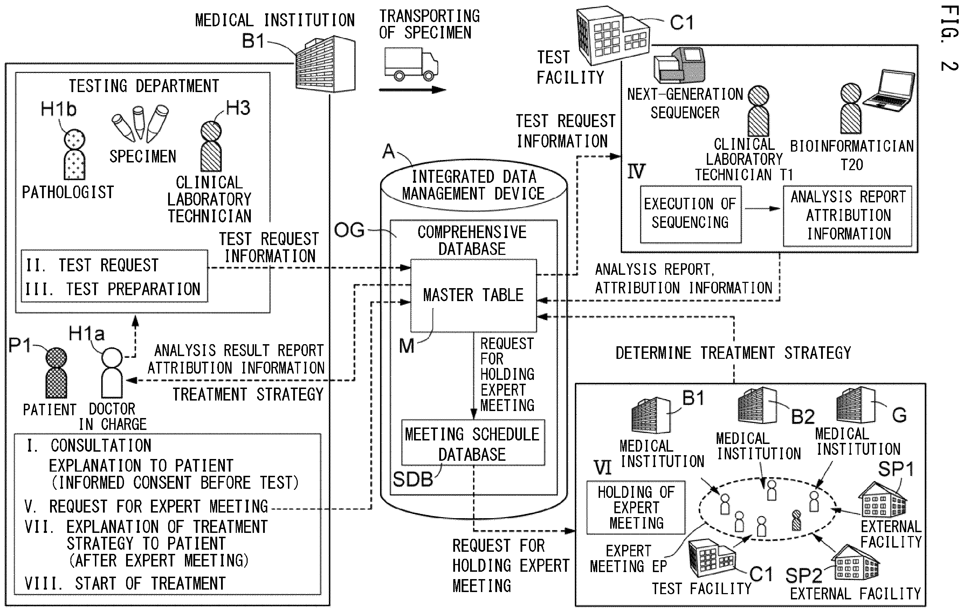

FIG. 2 shows a flow of the gene panel test according to the present embodiment. An exemplary case where the disease is cancer will be described.

The flow of the gene panel test in an exemplary case where the number of organizations is three will be described with reference to FIG. 2. A first organization that participates in the gene panel test is the medical institution B 1 such as a hospital consulted by a cancer patient. A second organization is the test facility C1 that performs the gene panel test. A third organization is an expert meeting EP. The three organizations can share information through an integrated data management device A for allowing the organizations to share information.

In the gene panel test (hereinafter, may be simply referred to as test), a patient P1 having a tumor consults the medical institution B1, and a doctor H1a in charge provides the patient P1 with explanation of contents, a flow, and the like of the gene panel test. In the explanation, informed consent is also obtained by, for example, confirming if the patient P1 and his/her family would like to know results including incidental findings such as germ cell mutation in a case where there are the incidental findings in the test (I in FIG. 2).

In a case where the patient P1 agrees to the execution of the gene panel test, the doctor H1a in charge makes a request for the gene panel test (II in FIG. 2). Information related to the test request is transmitted to the integrated data management device A, and stored together with patient information in a master table M that is stored in a comprehensive database OG. The information related to the test request includes information such as a test request date, test identification information (ID), a kind of the test panel, a kind of a specimen, and information (patient information) related to the patient. The patient information includes patient identification information (ID), sex, age, pathological diagnosis results, a medical history of the patient, a medical history of the patient's family, and the like.

By the test request having been made, a specimen of the patient required for the test is collected at the medical institution B1 (III in FIG. 2). Only a specimen that includes tumor cells is collected as the specimen from one patient, or both a specimen that includes tumor cells and a specimen that includes non-tumor cells are collected as the specimen from one patient. The specimen is collected by a pathologist H1b or a clinical laboratory technician H3. The collected specimen is transported to the test facility C1.

At the test facility C1, a clinical laboratory technician T1 performs pretreatment of the specimen, and performs sequencing of nucleic acid contained in the specimen by using a next-generation sequencer to execute the gene panel test (IV in FIG. 2). After the test has been executed, the clinical laboratory technician T1 and a bioinformatician T20 generate an analysis report (also referred to as report) in cooperation with each other.

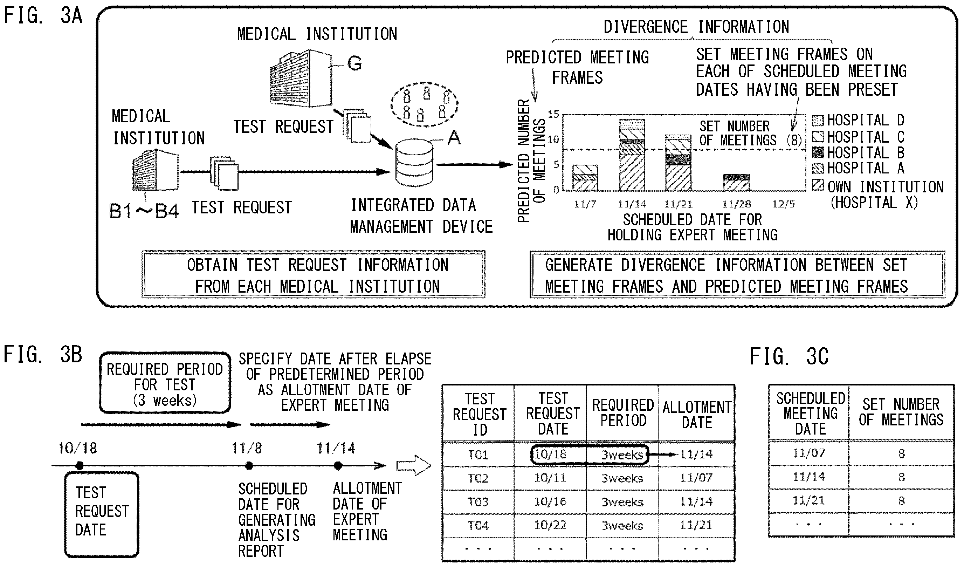

FIG. 3A shows an outline of the method for supporting management of the expert meetings according to embodiments of the present invention. With reference to FIG. 3A, the integrated data management device A obtains information related to the test requests for the gene panel tests from the medical institutions B1, B2, B3, B4 and the medical institution G. The integrated data management device A generates information of predicted meeting frames related to the number of meetings and/or a meeting time which are predicted on a scheduled date for holding the expert meeting, according to the obtained information of the requests for the gene panel tests. As shown in FIG. 3C, the scheduled dates for holding the expert meetings, and set meeting frames on each scheduled meeting date are determined in advance by a bureau of the expert meeting, and stored in the integrated data management device A. The integrated data management device A generates divergence information indicating a difference between the predicted meeting frames and the set meeting frames on each scheduled meeting date, such that the difference is recognizable. The divergence information is not particularly limited as long as the divergence information is represented in a display format that allows a user to recognize the difference between the predicted meeting frames and the set meeting frames.

The predicted meeting frames may represent the number of meetings (hereinafter, referred to as predicted number of meetings) predicted on the scheduled date for holding the expert meeting. The predicted meeting frames may represent the total of times of the meetings which is calculated from the predicted number of the meetings. For example, the predicted meeting frames can be obtained by multiplying an average meeting time per one meeting by the predicted number of the meetings. Alternatively, the predicted meeting frames can be obtained by predicting the meeting time for each of the meetings predicted to be held on the scheduled date for holding the expert meeting and calculating the total of all the meeting times.

The set meeting frames may represent the number of meetings (hereinafter, referred to as set number of meetings) set on the scheduled date for holding the expert meeting. The set meeting frames may represent the total of times of the meetings which is calculated from the set number of meetings. For example, the set meeting frames can be obtained by multiplying an average meeting time per one meeting by the set number of meetings. Alternatively, the set meeting frames can be obtained by predicting the meeting time for each of the meetings set on the scheduled date for holding the expert meeting and calculating the total of all the meeting times.

In order to generate information of the predicted meeting frames related to the number of meetings and/or the meeting time predicted on the scheduled date for holding the expert meeting, the scheduled date for holding the expert meeting (hereinafter, the scheduled date, for holding the expert meeting, which corresponds to the request information of each request is referred to as allotment date) in which the test result is deliberated on needs to be selected from a plurality of scheduled meeting dates according to the request information of the gene panel test. FIG. 3B shows a flow of selecting a scheduled meeting date, that is, an allotment date on which the test result is deliberated, from a plurality of scheduled meeting dates stored in the integrated data management device A, according to the request information of the gene panel test. A period from a day (October 18 in FIG. 3B) on which the request for the gene panel test is made to generation of an analysis report is estimated as three weeks. The integrated data management device A selects the scheduled date, for holding the expert meeting, after and closest to the scheduled date (November 8 in FIG. 3B) on which the analysis report is generated, as the allotment date (November 14 in FIG. 3B) for the test.

A tumor to be treated in genomic medicine is not limited. Examples of the tumor include benign epithelial tumor, benign nonepithelial tumor, malignant epithelial tumor, and malignant nonepithelial tumor. The origin of the tumor is not limited. Examples of the origin of the tumor include: tissues in a respiratory system such as a trachea, a bronchus, and a lung; tissues of a gastrointestinal tract such as a nasopharynx, an esophagus, a stomach, a duodenum, a jejunum, an ileum, a cecum, an appendix, an ascending colon, a transverse colon, a sigmoid colon, a rectum, and anus sites; a liver; a pancreas; tissues in a urinary system such as a bladder, a ureter, and a kidney; tissues in a female reproductive system such as an ovary, an oviduct, and a uterus; mammary gland; tissues in a male reproductive system such as prostate gland; skin; tissues in an endocrine system such as a hypothalamus, pituitary gland, thyroid gland, parathyroid gland, and adrenal gland; tissues in a central nervous system; bone and soft tissues; tissues in a hematopoietic system such as bone marrow and a lymph node; and blood vessels.

Genomic medicine is also applicable to diseases other than cancers.

The specimen is a sample containing nucleic acid derived from tumor cells, and examples thereof include tissues, body fluid, and excrements collected from a patient, and samples prepared therefrom. Examples of the body fluid include blood, bone marrow aspirate, ascitic fluid, pleural fluid, and spinal fluid. Examples of the excrement include feces and urine. A liquid such as intraperitoneal washing liquid or large intestine washing liquid obtained after washing a part of a body of a patient may be used.

### II. Support system

### 1. Configuration of system

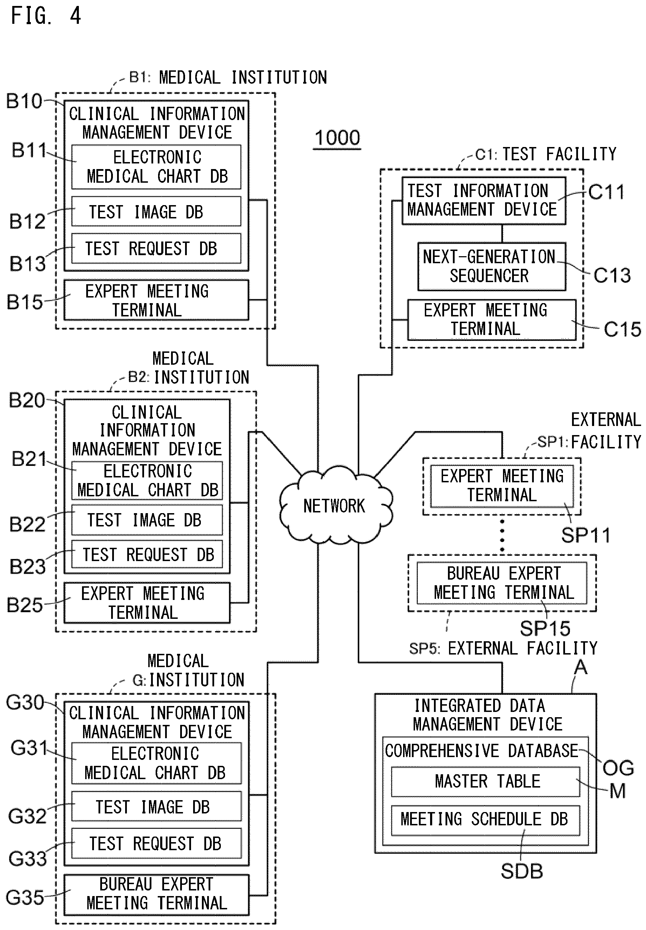

A configuration of a system 1000 (hereinafter, simply referred to as system 1000) for supporting, by using a computer, management of the expert meeting in which a plurality of medical workers interpret gene information of a patient will be described with reference to FIG. 4. The system 1000 can include one computer or a plurality of computers. The system 1000 includes the integrated data management device A, a clinical information management device B10 and an expert meeting terminal B15 disposed in the medical institution B1, a clinical information management device B20 and an expert meeting terminal B25 disposed in the medical institution B2, a clinical information management device G30 and a bureau expert meeting terminal G35 disposed in the medical institution G, a test information management device C11, a next-generation sequencer C13 connected to the test information management device C11, an expert meeting terminal C15 in the test facility C1, and an expert meeting terminal SP11 disposed in an external facility SP1. The clinical information management device B10, the expert meeting terminal B 15, the clinical information management device B20, the expert meeting terminal B25, the clinical information management device G30, and the bureau expert meeting terminal G35 are connected via a wired or wireless network so as to be communicable with the integrated data management device A. The clinical information management devices B10, B20, G30 are each a management device that integrally manages information, such as a test request, test results, prescription information, meal information, and surgery information, described in a medical chart, in the medical institution. The clinical information management devices B10, B20, G30 store electronic medical chart databases (electronic medical chart DB) B11, B21, G31, test image databases (test image DB) B12, B22, G32, and test request databases (test request DB) B 13, B23, G33, respectively. The expert meeting terminal B15, B25 in each medical institution displays a graphical user interface (GUI) outputted by the integrated data management device A, and is used when a doctor in charge in each medical institution participates in the expert meeting. The expert meeting terminal G35 is a terminal used by the expert meeting bureau that manages the expert meeting. In the present embodiment, a dedicated application for accessing the integrated data management device A is installed in each of the clinical information management device B10, the expert meeting terminal B15, the clinical information management device B20, the expert meeting terminal B25, the clinical information management device G30, and the bureau expert meeting terminal G35. Instead of the dedicated application for accessing the integrated data management device A, a general-purpose web browser such as Internet Explorer and Google Chrome can be used.

The test information management device C11 and the expert meeting terminal C15 in the test facility C1 are connected via a wired or wireless network so as to be communicable with the integrated data management device A. The test information management device C11 analyzes a nucleic acid sequence by using nucleic acid sequence data obtained from the next-generation sequencer C13. Furthermore, for example, the test information management device C11 manages acceptance of a specimen, quality information of the specimen and the test, and a test progress state. The expert meeting terminal C15 in the test facility C1 displays a graphical user interface (GUI) outputted by the integrated data management device A, and is used by a clinical laboratory technician and a bioinformatician who participate in the expert meeting when they participate in the expert meeting.

In a case where the medical institution G commits management of the expert meetings to an external facility, the system 1000 may further include the expert meeting terminal SP11 disposed in an external facility SP1 and an expert meeting terminal SP15 in the external facility. The expert meeting terminal SP11 in the external facility SP1 is connected via a wired or wireless network so as to be communicable with the integrated data management device A. The expert meeting terminal SP11 displays a graphical user interface (GUI) outputted by the integrated data management device A, and is used by a genetic counselor and a molecular genetics researcher who participate in the expert meeting when they participate in the expert meeting. The number of the external facilities may be plural. In the description herein, an exemplary case where five external facilities represented by SP1 to SP5 are present will be described. The expert meeting terminals disposed in the external facilities are also represented as the external facility expert meeting terminals SP1 1 to SP15. One of the external facility expert meeting terminals, for example, the expert meeting terminal SP15 may be a terminal to be used by the expert meeting bureau that manages the expert meetings. The external facility expert meeting terminal SP15 is also referred to as a bureau expert meeting terminal SP15, and is used for registering a new expert meeting schedule frame in an expert meeting schedule database SDB.

In the system 1000, the integrated data management device A integrates the test request information, analysis results, the expert meeting setting, and the like related to the gene panel test. Therefore, the other devices and terminals may be referred to as "other computers" in embodiments.

### 2. Integrated data management device

### 2-1. Hardware configuration of integrated data management device

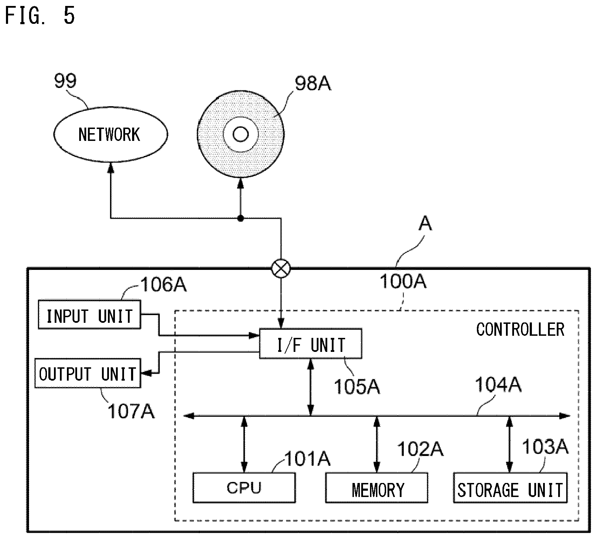

The integrated data management device A functions as a support apparatus A for supporting, by using a computer, management of the expert meeting in which the plurality of medical workers interpret gene information of a patient. FIG. 5 shows a hardware configuration of the integrated data management device A (simply referred to also as "management device A").

The integrated data management device A is a general-purpose computer.

The integrated data management device A includes a controller 100A, an input unit 106A, and an output unit 107A.

The controller 100A includes a CPU (central processing unit) 101A for performing data processing described below, a memory 102A used as a temporary storage region for performing the data processing, a storage unit 103A for storing process data and programs described below, and a bus 104A that allows transmission of data among the units and the like. The input unit 106A and the output unit 107A are connected to the controller 100A. For example, the input unit 106A includes a keyboard, a mouse, a touch sensor, and the like. The output unit 107A includes a display, a printer, a speaker, and the like. Alternatively, a device, such as a touch panel having a touch sensor and a display integrated with each other, having functions of both the input unit and the output unit may be used. An I/F unit 105A is an interface that allows the controller 100A to communicate with an external device or a network. The controller 100A is connected to a network 99 via the I/F unit 105A, and can communicate with the clinical information management device B10, the expert meeting terminal B15 in the medical institution B1, the clinical information management device B20, the expert meeting terminal B25 in the medical institution B2, the clinical information management device G30, the bureau expert meeting terminal G35 in the medical institution G, the test information management device C11, the expert meeting terminal C15 in the test facility C1, the expert meeting terminal SP11 in the external facility, and the expert meeting terminal SP15 in the external facility.

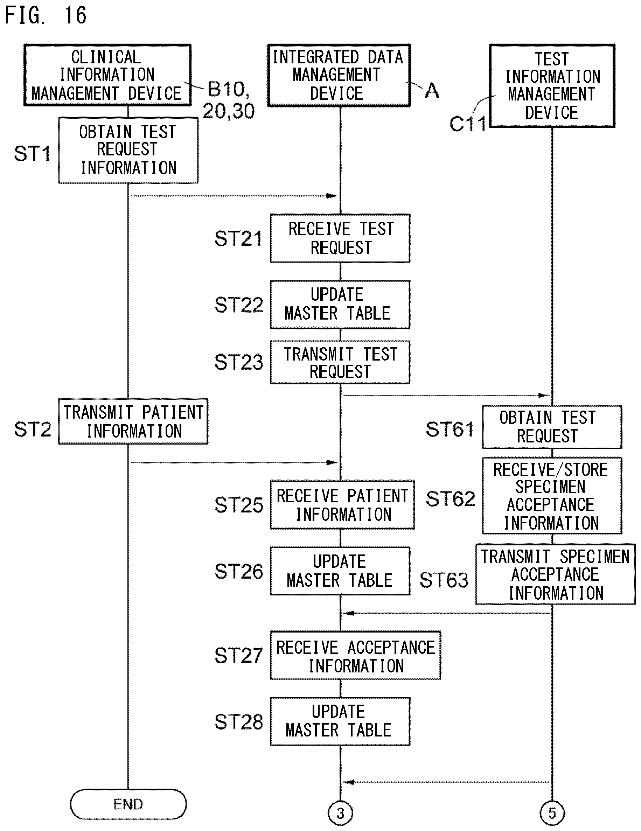

The storage unit 103A previously stores an operating system (OS), application programs for performing processes in steps shown below in FIG. 16, FIG. 17, FIG. 19, FIG. 20, FIG. 21, FIG. 24, FIG. 25, FIG. 27, FIG. 30, FIG. 39, and FIG. 41, and mail software, in the storage unit 103A in, for example, an executable form. The executable form is, for example, a form generated through conversion from a programming language by a compiler. The controller 100A uses each of the programs stored in the storage unit 103A to perform each of the processes shown in FIG. 16, FIG. 17, FIG. 19, FIG. 20, FIG. 21, FIG. 24, FIG. 25, FIG. 27, FIG. 30, FIG. 39, and FIG. 41. The storage unit 103A stores the comprehensive database OG that stores various databases which are used for processes described below and which include the master table M, the expert meeting schedule database SDB linked to the master table M, and the like. The controller 100A updates information in the master table M based on information transmitted from each clinical information management device, the test information management device, and the expert meeting terminal in each of the medical institutions. Furthermore, the controller 100A updates information in the expert meeting schedule database SDB based on information transmitted from the expert meeting terminal in each of the medical institutions, the expert meeting terminal in the test facility C1, and the expert meeting terminal in each of the external facilities.

In the following description, unless otherwise specified, the processes performed by the controller 100A are processes performed by the CPU 101A based on the application programs stored in the storage unit 103A or the memory 102A. The CPU 101A temporarily stores necessary data (intermediate data being processed, or the like) in the memory 102A as a work area in a volatile manner, and stores, in the storage unit 103A, data such as analysis results to be stored for a long time in a non-volatile manner as appropriate.

The application program may be downloaded from an external storage medium 98A such as a DVD or a USB memory, and installed in the storage unit 103A of the controller 100A.

### 2-2. Functional configuration of controller of integrated data management device

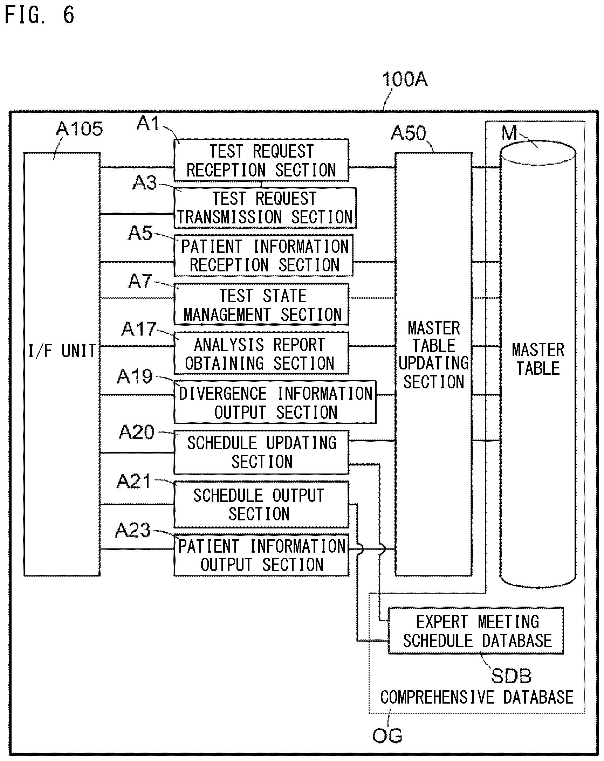

FIG. 6 shows a functional configuration of the controller 100A of the integrated data management device A.

The controller 100A of the integrated data management device A includes a test request reception section A1, a test request transmission section A3, a patient information reception section A5, a test state management section A7, an analysis report obtaining section A17, a divergence information output section A19, a schedule updating section A20, a schedule output section A21, a patient information output section A23, a master table updating section A50, and the comprehensive database OG. The comprehensive database OG includes the master table M and the expert meeting schedule database SDB.

The master table updating section A50 updates information in the master table M according to information received by the test request reception section A1, the test request transmission section A3, the patient information reception section A5, the test state management section A7, the analysis report obtaining section A17, the divergence information output section A19, the schedule updating section A20, and the patient information output section A23. The schedule updating section A20 stores, in the expert meeting schedule database SDB, information related to a meeting schedule inputted by the bureau for holding the expert meeting.

The schedule output section A21 transmits date and time set for the expert meeting, to the expert meeting terminal B15, B25, G35, C15, SP11, SP15 in each of the medical institutions and facilities by using the mail software or the like. The mail transmission destinations are stored in the comprehensive database OG so as to be associated with the bureau ID in, for example, the master table M.

### 2-3. Structure of master table

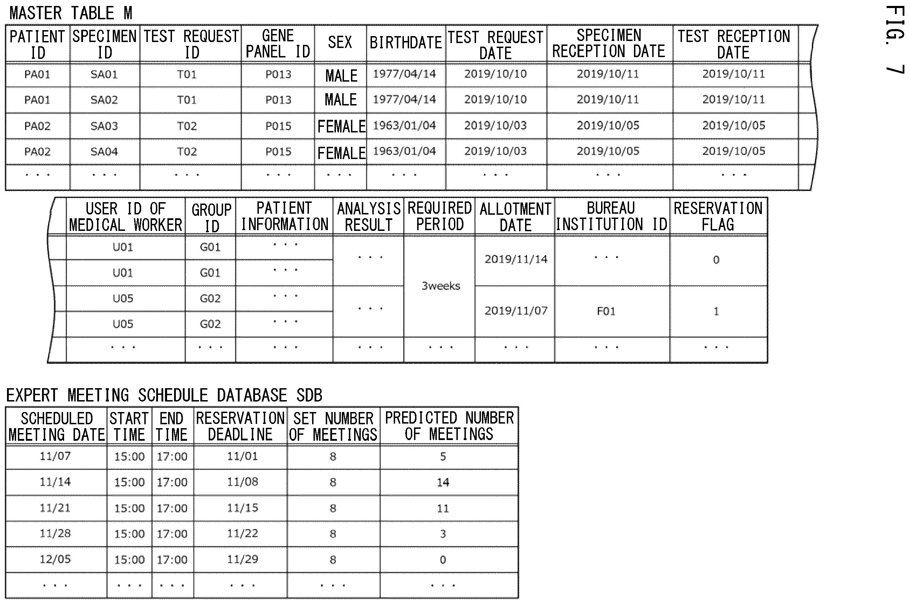

FIG. 7 shows examples of the master table M and the expert meeting schedule database SDB.

The master table M includes a region in which a "patient ID" is stored as an identification label of a patient, a region in which a "specimen ID" is stored as an identification label of a specimen, a region in which a "test request ID" is stored as an identification label of a test request, a region in which a "gene panel ID" is stored as an identification label of a gene panel test, a region in which a "sex of patient" is stored, a region in which a "birthdate of patient" is stored, a region in which a "test request date" is stored, a region in which a "specimen reception date" is stored, a region in which a "test reception date" is stored, a region in which a "user ID of medical worker" is stored as an identification label of a doctor in charge, a region in which a "group ID" is stored as a group label of a group in charge of the expert meeting, a region for indicating "patient information" as information related to clinical information for the patient, a region in which an "analysis result" is stored as information related to results of the gene panel test, a region in which a "required time" is stored as information related to a required time from a test reception date to generation of analysis results, a region in which an "allotment date" is stored as information related to an allotment date of the expert meeting for the patient identified by the patient ID in the same row, a region in which a "bureau institution ID" is stored as an identification label of the bureau of the expert meeting, and a region in which a "reservation flag" is stored as information indicating whether or not a reservation for holing a meeting on the allotment date has been made.

The expert meeting schedule database SDB includes a region in which a "scheduled meeting date" is stored as information of a scheduled date for holding the expert meeting, a region in which a "start time" is stored as information of a start time of the meeting on each scheduled meeting date, a region in which an "end time" is stored as information of an end time of the meeting on each scheduled meeting date, a region in which a "reservation deadline" is stored as information of a reservation deadline for the meeting on each scheduled meeting date, a region in which the "number of meeting frames" is stored as information of the number of meeting frames on each scheduled meeting date, and a region in which the "predicted number of frames" is stored as information of the number of the meetings predicted to be held on each scheduled meeting date. The number of the meetings on the date, the total of times of the meetings on the date, or both of them may be stored in the "number of meeting frames" region.

FIG. 7 shows an example of the master table M for a case where a first specimen containing tumor cells and a second specimen containing normal cells are used as a specimen in one test for one patient in the gene panel test. Two kinds of specimens are used in one test for one patient. Therefore, the same contents are indicated in the first and the second lines, and the same contents are indicated in the third and the fourth lines in the master table M shown in FIG. 7 except for the "specimen ID".

### 3. Clinical information management device

### 3-1. Hardware configuration of clinical information management device

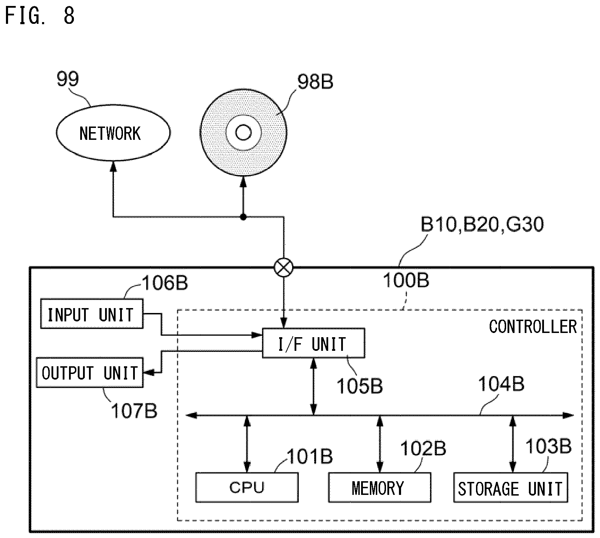

FIG. 8 shows a hardware configuration of the clinical information management device B10, B20, G30.

The clinical information management device B10, B20, G30 is a general-purpose computer. The clinical information management devices B10, B20, G30 each have the same hardware configuration as the integrated data management device A. A controller 100B, an input unit 106B, an output unit 107B, a CPU 101B, a memory 102B, a storage unit 103B, a bus 104B, and an I/F unit 105B for each of the clinical information management devices B10, B20, G30 correspond to the controller 100A, the input unit 106A, the output unit 107A, the CPU 101A, the memory 102A, the storage unit 103A, the bus 104A, and the I/F unit 105A, respectively, in the integrated data management device A.

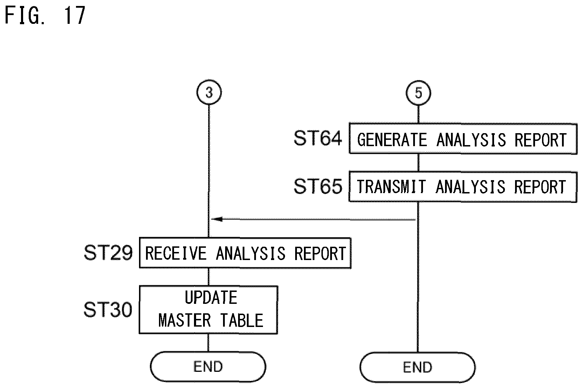

The storage unit 103B previously stores, for example, an operating system (OS), a computer program for performing the process in each step shown below in FIG. 16 and FIG. 17, a computer program for displaying an electronic medical chart stored in the electronic medical chart database (DB) B11, a computer program for displaying test images stored in the test image database (DB) B12, a computer program for performing a test request at a hospital or the like, browser software for performing the test request for the gene panel test, and browser software for displaying, for example, display information outputted by the integrated data management device A. The storage unit 103B stores the electronic medical chart database (DB) B11, the test image database (DB) B12, and the test request database (DB) B13.

The above-described computer programs and browser software may be downloaded from an external storage medium 98B such as a DVD or a USB memory, and installed in the storage unit 103B.

The controller 100B is connected to the network 99 via the I/F unit 105B and communicates with the integrated data management device A.

### 3-2. Functional configuration of controller of clinical information management device

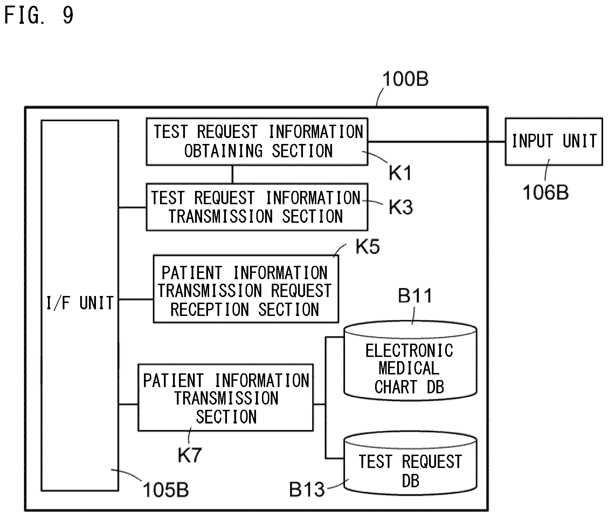

FIG. 9 shows a functional configuration of the controller 100B of the clinical information management device B10, B20, G30.

The controller 100B of the clinical information management device B10, B20, G30 includes a test request information obtaining section K1, a test request information transmission section K3, a patient information transmission request reception section K5, a patient information transmission section K7, the electronic medical chart database (DB) B11, the test image database (DB) B12, and the test request database (DB) B13.

The test request information obtaining section K1 receives information related to a test request, from a user of the clinical information management device B10, B20, G30. The test request information transmission section K3 transmits the information related to the test request, to the integrated data management device A. The patient information transmission request reception section K5 receives a transmission request for transmitting information related to a patient, from a user of the clinical information management device B10, B20, G3. The patient information transmission section K7 reads patient information from the electronic medical chart database (DB) B11 and/or the test image database (DB) B12, and transmits the read patient information to the integrated data management device A.

### 4. Test information management device

### 4-1. Hardware configuration of test information management device

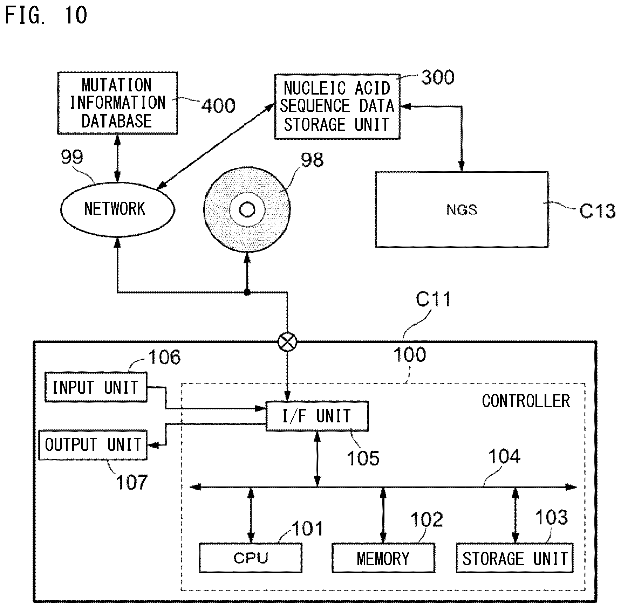

FIG. 10 shows a hardware configuration of the test information management device C11. The test information management device C11 is a general-purpose computer. The test information management device C11 has the same hardware configuration as the integrated data management device A.

The test information management device C11 includes a controller 100, an input unit 106, and an output unit 107.

The controller 100 includes a CPU 101 for performing data processing described below, a memory 102 used as a temporary storage region for performing the data processing, a storage unit 103 for storing process data and a program described below, a bus 104 that allows transmission of data among the units and the like, and an I/F unit 105 for performing data input and output with external devices. The input unit 106 and the output unit 107 are connected to the controller 100. For example, the input unit 106 includes a keyboard, a mouse, a touch sensor, and the like. The output unit 107 includes a display, a printer, a speaker, and the like. Alternatively, a device, such as a touch panel having a touch sensor and a display integrated with each other, having functions of both the input unit and the output unit may be used. The I/F unit 105 is an interface that allows the controller 100 to communicate with an external device.

The storage unit 103 of the controller 100 previously stores an operating system and an application program for performing the process in each step shown below in FIG. 16, in the storage unit 103 in, for example, an executable form. The executable form is, for example, a form generated through conversion from a programming language by a compiler. The controller 100 uses the program stored in the storage unit 103 to perform a nucleic acid sequence analyzing process and an attribution information obtaining process.

The test information management device C11 can be connected via the network 99 to a mutation information database 400 and a nucleic acid sequence data storage unit 300.

The nucleic acid sequence data storage unit 300 is a computer for storing nucleic acid sequence data obtained by the next-generation sequencer C13.

### 4-2. Functional configuration of controller of test information management device

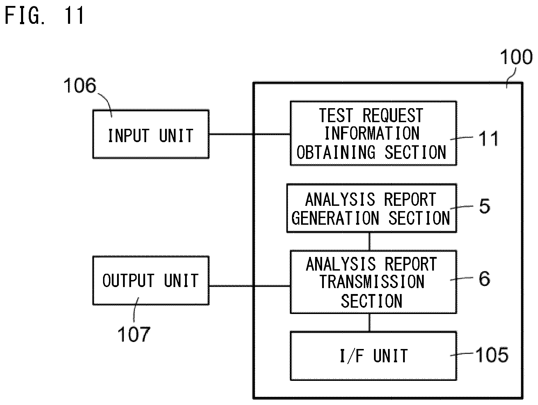

FIG. 11 shows a functional configuration of the controller 100 of the test information management device C11.

The controller 100 of the test information management device C11 includes a test request information obtaining section 11, an analysis report generation section 5, and an analysis report transmission section 6.

The test request information obtaining section 11 obtains information related to a test request, from the integrated data management device A. The analysis report generation section 5 obtains nucleic acid sequence data obtained from each of specimens, from the nucleic acid sequence data storage unit 300, and generates an analysis report. The analysis report transmission section 6 transmits the analysis report generated by the analysis report generation section 5, to the integrated data management device A.

### 5. Expert meeting terminal in medical institution

### 5-1. Hardware configuration of expert meeting terminal in medical institution

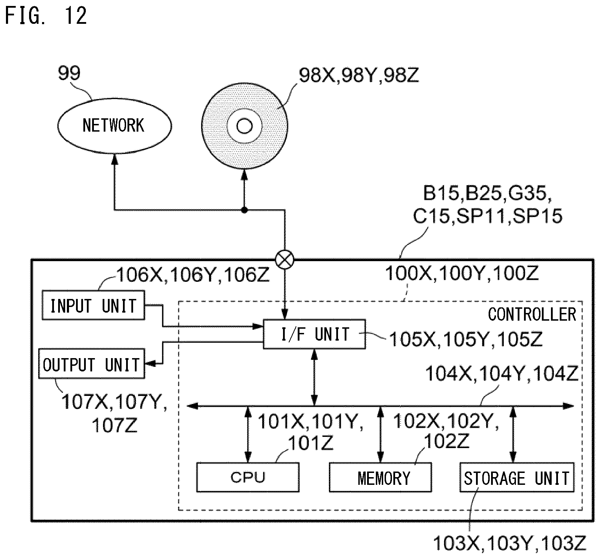

FIG. 12 shows a hardware configuration of the expert meeting terminal B15, B25, G35 disposed in the medical institution B1, B2, G

The expert meeting terminal B 15, B25, G35 disposed in the medical institution B1, B2, G is a general-purpose computer. The expert meeting terminals B15, B25, G35 each have the same hardware configuration as the integrated data management device A. A controller 100X, an input unit 106X, an output unit 107X, a CPU 101X, a memory 102X, a storage unit 103X, a bus 104X, and an I/F unit 105X for each of the expert meeting terminals B15, B25, G35 correspond to the controller 100A, the input unit 106A, the output unit 107A, the CPU 101A, the memory 102A, the storage unit 103A, the bus 104A, and the I/F unit 105A, respectively, in the integrated data management device A.

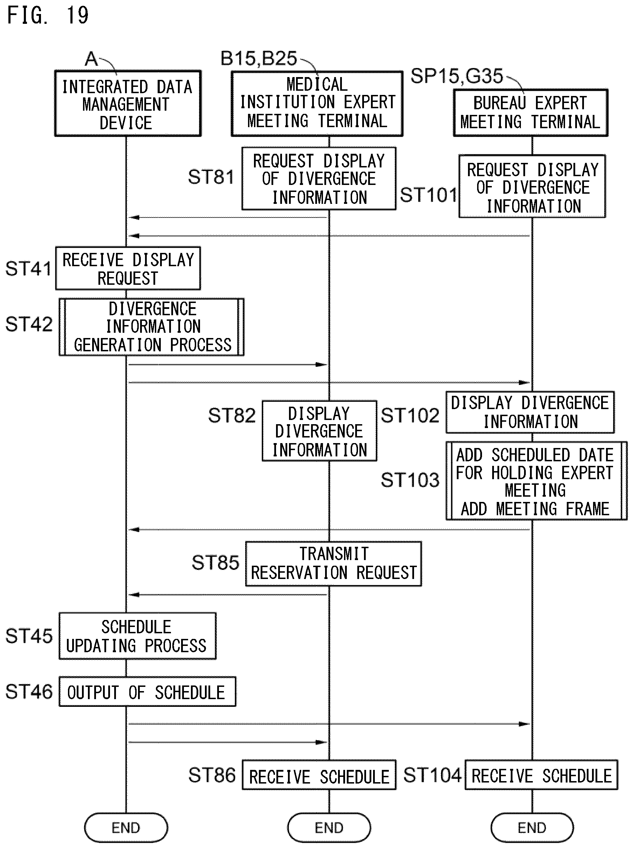

The storage unit 103X previously stores, for example, an operating system (OS), a computer program for performing the process in each step shown below in FIG. 19, and browser software for displaying, for example, display information outputted by the integrated data management device A.

The browser software may be downloaded from an external storage medium 98X such as a DVD or a USB memory, and installed in the storage unit 103X.

The controller 100X is connected via the I/F unit 105X to the network 99 and communicates with the integrated data management device A.

### 5-2. Functional configuration of controller of expert meeting terminal in medical institution

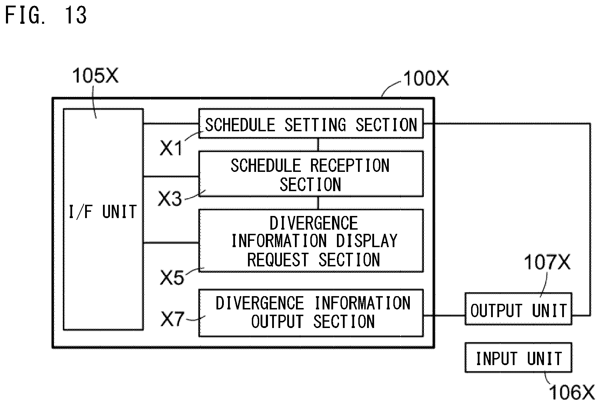

FIG. 13 shows a functional configuration of the controller 100X of the expert meeting terminal B15, B25, G35 disposed in the medical institution B1, B2, G.

The controller 100X of the expert meeting terminal B15, B25, G35 disposed in the medical institution B1, B2, G includes a schedule setting section X1, a schedule reception section X3, a divergence information display request section X5, and a divergence information output section X7.

### 6. Expert meeting terminal in test facility and expert meeting terminal in external facility

### 6-1. Hardware configurations of expert meeting terminal in test facility and expert meeting terminal in external facility

FIG. 12 shows a hardware configuration of each of the expert meeting terminal C15 in the test facility C1 and the expert meeting terminal SP11 in the external facility SP1.

The expert meeting terminal C15 and the expert meeting terminal SP11 are each a general-purpose computer. The expert meeting terminal C15 and the expert meeting terminal SP11 each have the same hardware configuration as the integrated data management device A. A controller 100Y, an input unit 106Y, an output unit 107Y, a CPU 101Y, a memory 102Y, a storage unit 103Y, a bus 104Y, and an I/F unit 105Y for each of the expert meeting terminal C15 in the test facility C1 and the expert meeting terminal SP11 in the external facility SP1 correspond to the controller 100A, the input unit 106A, the output unit 107A, the CPU 101A, the memory 102A, the storage unit 103A, the bus 104A, and the I/F unit 105A, respectively, in the integrated data management device A.

The storage unit 103Y previously stores, for example, an operating system (OS), a computer program for performing the process in each step shown below in FIG. 19, and browser software for displaying, for example, display information outputted by the integrated data management device A.

The browser software may be downloaded from an external storage medium 98Y such as a DVD or a USB memory, and installed in the storage unit 103Y.

The controller 100Y is connected via the I/F unit 105Y to the network 99 and communicates with the integrated data management device A.

### 6-2. Functional configuration of controller of each of expert meeting terminal in test facility and expert meeting terminal in external facility

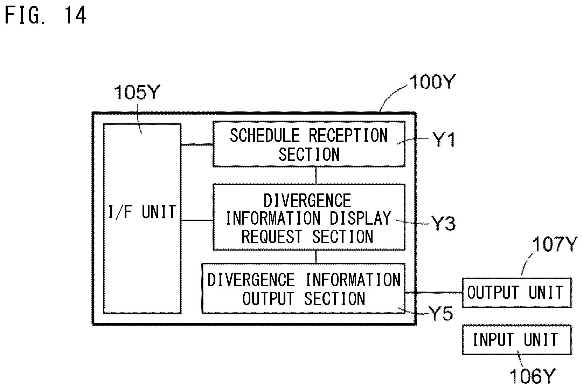

FIG. 14 shows functional configurations of the controller 100Y of the expert meeting terminal C15 in the test facility C1 and the controller 100Y of the expert meeting terminal SP11 in the external facility SP1.

The controller 100Y of each of the expert meeting terminal C15 and the expert meeting terminal SP11 includes a schedule reception section Y1, a divergence information display request section Y3, and a divergence information output section Y5.

### 7. Bureau expert meeting terminal

### 7-1. Hardware configuration of bureau expert meeting terminal

FIG. 12 shows a hardware configuration of the bureau expert meeting terminal G35, SP15.

The bureau expert meeting terminal G35, SP15 is a general-purpose computer. The bureau expert meeting terminals G35, SP15 each have the same hardware configuration as the integrated data management device A. A controller 100Z, an input unit 106Z, an output unit 107Z, a CPU 101Z, a memory 102Z, a storage unit 103Z, a bus 104Z, and an I/F unit 105Z for each of the bureau expert meeting terminals G35, SP15 correspond to the controller 100A, the input unit 106A, the output unit 107A, the CPU 101A, the memory 102A, the storage unit 103A, the bus 104A, and the I/F unit 105A, respectively, in the integrated data management device A.

The storage unit 103Z previously stores, for example, an operating system (OS), a computer program for performing the process in each step shown below in FIG. 19, and browser software for displaying, for example, display information outputted by the integrated data management device A.

The browser software may be downloaded from an external storage medium 98Z such as a DVD or a USB memory, and installed in the storage unit 103Z.

The controller 100Z is connected via the I/F unit 105Z to the network 99, and communicates with the integrated data management device A.

### 7-2. Functional configuration of controller of bureau expert meeting terminal

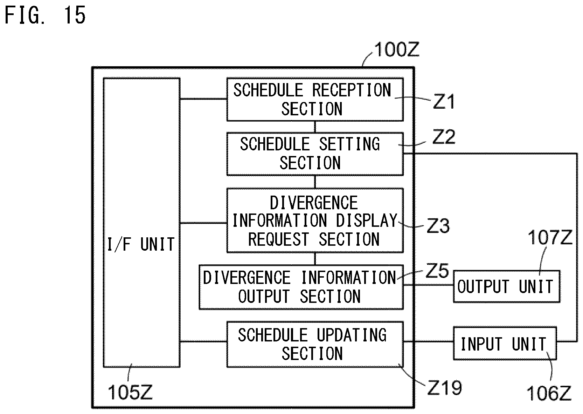

FIG. 15 shows a functional configuration of the controller 100Z of the bureau expert meeting terminal G35, SP15.

The controller 100Z of the bureau expert meeting terminal G35, SP15 includes a schedule reception section Z1, a schedule setting section Z2, a divergence information display request section Z3, a divergence information output section Z5, and a schedule updating section Z19.

### 8. Operation of system

An operation performed by the system 1000 for making a test request for the gene panel test will be described with reference to FIG. 16 to FIG. 19.

### 8-1. Flow of test request

In the system 1000, a request for the gene panel test for a patient having a tumor is firstly made by the medical institution B 1, B2, G.

The number of the medical institutions that participate in the system 1000 may be plural. In the description herein, the operation will be described by using the medical institution B1 as an example. In this example, the clinical information management device B10 and the expert meeting terminal B15 in the medical institution B1 are used.

The controller 100B (hereinafter, simply referred to as the clinical information management device B10) of the clinical information management device B10 disposed in the medical institution B1 receives input of test request information from the doctor H1a in charge through the input unit 106B in step ST1 in FIG. 16. At this time, the controller 100B functions as the test request information obtaining section K1 shown in FIG. 9. The process performed by the test request information obtaining section K1 will be described below.

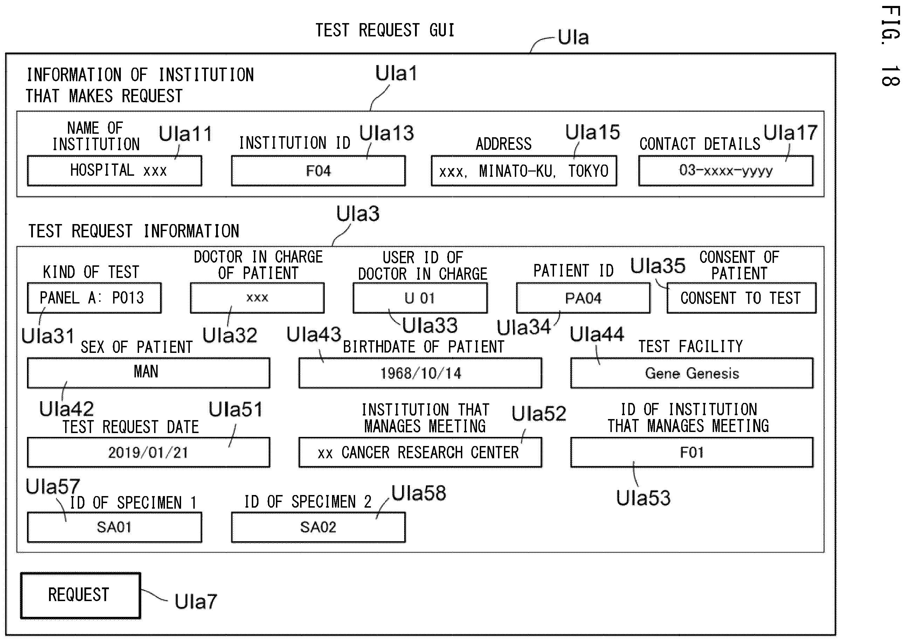

The test request is inputted via a user interface UIa shown in FIG. 18. The user interface UIa may include a medical institution information input region UIa1 for inputting information of a medical institution that makes a request, a test request information input region UIa3 for inputting the test request information, and a request determination icon UIa7 for determining the request. The medical institution information input region UIa1 includes a region UIa11 for displaying a name of the institution, a region UIa13 for inputting identification information (ID) of the institution, a region UIa1 5 for inputting an address of the institution, and a region UIa17 for inputting contact details of the institution.

The test request information input region UIa3 includes a region UIa31 for inputting a kind of a test for specifying a requested gene panel test, a region UIa32 for inputting a name of a doctor in charge of a patient for whom the test is requested, a region UIa33 for inputting identification information of the doctor in charge as a user in the gene panel test, a region UIa34 for inputting identification information (ID) of the patient, a region UIa35 for inputting information related to informed consent of the patient, a region UIa42 for inputting a sex of the patient, a region UIa43 for inputting a birthdate of the patient, a region UIa44 for inputting a name of a test facility that is requested to perform the gene panel test, a region UIa51 for inputting a test request date, a region UIa52 for inputting a name of an institution serving as a bureau that manages the expert meeting, a region UIa53 for inputting identification information (ID) of the institution that serves as the bureau, a region UIa57 for inputting an ID of the first specimen containing nucleic acid derived from tumor cells, and a region UIa58 for inputting an ID of the second specimen containing nucleic acid derived from non-tumor cells.

When the doctor H1a in charge inputs a part or the entirety of the regions in the user interface UIa through the input unit 106B of the clinical information management device B10, and selects the request determination icon UIa7, the clinical information management device B10 transmits the contents inputted in the user interface UIa as information related to the test request, to the integrated data management device A. At this time, the controller 100B of the clinical information management device B10 functions as the test request information transmission section K3.

The controller 100A (hereinafter, simply referred to as the integrated data management device A) of the integrated data management device A receives the test request information transmitted from the clinical information management device B10, via the I/F unit 105A, in step ST21 in FIG. 16. At this time, the controller 100A functions as the test request reception section A1.

Subsequently, in step ST22, the integrated data management device A stores the test request information in the master table M, and updates the master table M. At this time, the controller 100A functions as the master table updating section A50.

In the process of updating the master table M in step ST22 in FIG. 16, for example, the test-request-related information having been inputted through the user interface UIa can be reflected in the master table M in the following correspondence.

The patient ID input region UIa34 is reflected in a column representing the "patient ID" in the master table M.

The first specimen ID input region UIa57 and the second specimen ID input region UIa58 are reflected in a column representing the "specimen ID" in the master table M.

The test kind input region UIa31 is reflected in the "gene panel ID" region in the master table M.

The patient sex input region UIa42 is reflected in the "sex of patient" region in the master table M.

The patient birthdate input region UIa43 is reflected in the "birthdate of patient" region in the master table M.

The test request date input region UIa51 is reflected in the "test request date" region in the master table M.

The doctor-in-charge user ID input region UIa33 is reflected in the "user ID of medical worker" region in the master table M.

The bureau institution name input region UIa52 is reflected in the "bureau institution" region in the master table M.

Test-request-related information, other than the above-described information, for which input regions are not indicated in FIG. 7 is also stored in a predetermined region in the master table M.

Next, the integrated data management device A transmits the test request information obtained in step ST22 via the I/F unit 105A to the test information management device C11 in step ST23 in FIG. 16. In step ST23, the controller 100A of the integrated data management device A functions as the test request transmission section A3. The transmission in step ST23 in FIG. 16 may be triggered by updating the master table M by the integrated data management device A or triggered by inputting a transmission request through the input unit 106A by an operator who operates the integrated data management device A.

The controller 100 (hereinafter, simply referred to also as the test information management device C11) of the test information management device C11 obtains the test request information transmitted from the integrated data management device A, via the I/F unit 105, in step ST61. In step ST61, the test information management device C11 stores the obtained test request information in the storage unit 103. At this time, the controller 100 of the test information management device C11 functions as the test request information obtaining section 11.

Next, the clinical information management device B10 reads and obtains patient information of the patient for which the test request has been made, from the electronic medical chart database B11 and/or the test image database B12, and transmits the patient information to the integrated data management device A in step ST2 in FIG. 16. At this time, the controller 100B of the clinical information management device B10 functions as the patient information transmission section K7. The information to be transmitted in step ST2 in FIG. 16 may be transmitted so as be included in the test request information when the test request information is transmitted in step ST1. The transmission of the patient information may be triggered by inputting a transmission request through the input unit 106B to the controller 100B by the doctor H1a in charge. At this time, the controller 100B of the clinical information management device B10 functions as the patient information transmission request reception section K5 (FIG. 9).

The integrated data management device A receives the patient information via the I/F unit 105A in step ST25 in FIG. 16. At this time, the controller 100A of the integrated data management device A functions as the patient information reception section A5. In step ST26 in FIG. 16, the integrated data management device A stores the patient information in the master table M and updates the master table M. The patient information is stored in the "patient information" region in the master table M. At this time, the controller 100A of the integrated data management device A functions as the master table updating section A50 (FIG. 6).

The process steps of step ST2, step ST25, and step ST26 in FIG. 16 may be performed before step ST1 in FIG. 16.

### 8-2. Flow of gene panel test

The gene panel test is started by receiving specimen reception acceptance information by the test information management device C11. By transporting a specimen of the patient collected in the medical institution B 1 to the test facility C1, for example, the clinical laboratory technician T1 of the test facility C1 inputs a label for indicating that the specimen has been accepted, to the storage unit 103, through the input unit 106 of the test information management device C11. In step ST62 in FIG. 16, the test information management device C11 receives the input.

In step ST63 in FIG. 16, the test information management device C11 transmits the label indicating that the specimen received in step ST62 has been accepted, as specimen acceptance information, to the integrated data management device A.

In step ST27, the integrated data management device A receives the specimen acceptance information transmitted by the test information management device C11 in step ST63. At this time, the controller 100A of the integrated data management device A functions as the test state management section A7. In step ST28, the integrated data management device A updates the "specimen reception date" region in the master table M, based on the contents received in step ST27. At this time, the controller 100A of the integrated data management device A functions as the master table updating section A50 (FIG. 9). The transmission of the information in step ST63 in FIG. 16 may be triggered by receiving the specimen acceptance information by the test information management device C11 or by inputting a transmission request through the input unit 106 by the clinical laboratory technician T1 or the like.

The test information management device C11 generates an analysis report of the gene panel test based on nucleic acid sequence data obtained from a sequence of the patient, in step ST64 in FIG. 17. At this time, the controller 100 of the test information management device C11 functions as the analysis report generation section 5 (FIG. 11). The test information management device C11 transmits the generated analysis report to the integrated data management device A in step ST65. At this time, the controller 100 of the test information management device C11 functions as the analysis report transmission section 6 (FIG. 11). The transmission of the analysis report in step ST65 may be triggered by the analysis report having been generated. Alternatively, the transmission of the analysis report may be triggered by inputting a transmission request through the input unit 106 by the clinical laboratory technician T1 or the like, and receiving the transmission request by the test information management device C11.

The integrated data management device A receives, in step ST29, the analysis result report transmitted in step ST65 in FIG. 17, and stores the analysis result report in the "analysis result" region in the master table M and updates the master table M in step ST30.

Through the processes in step ST21 to step ST28 in FIG. 16 and step ST29 to step ST30 in FIG. 17, the integrated data management device A obtains the information related to the test request from the clinical information management device B10, B20, G30, and further obtains the information related to the specimen reception date and the analysis results from the test information management device C11, and the integrated data management device A integrates the information and the like with each other, and stores the information and the like in the master table M shown in FIG. 7.

### 8-3. Divergence information display process

Next, a divergence information display process will be described with reference to FIG. 19.

In the divergence information display process, the integrated data management device A and the expert meeting terminal B15, B25 in the medical institution B1, B2 communicate with each other. Furthermore, the integrated data management device A and the bureau expert meeting terminal G35 communicate with each other. In a case where the medical institution G commits management of the expert meetings to the external facility, the integrated data management device A and the bureau expert meeting terminal SP15 communicate with each other.

A flow of a process performed between the integrated data management device A and the expert meeting terminal B 15, B25 in the medical institution B 1, B2 will be firstly described. The process performed by the expert meeting terminal B15 in the medical institution B1 and the process performed by the expert meeting terminal B25 in the medical institution B2 are the same. Therefore, the flow of the process performed by the expert meeting terminal B15 in the medical institution B 1 will be described.

The doctor H1a in charge accesses the integrated data management device A via the browser software from the medical institution B1 expert meeting terminal B 15 disposed in the medical institution B1. In step ST81 in FIG. 19, the controller 100X (hereinafter, simply referred to as "the expert meeting terminal B15 in the medical institution B1") of the expert meeting terminal B 15 in the medical institution B 1 receives a request for displaying the divergence information, through the input unit 106X, from the doctor H1a in charge. Subsequently, the expert meeting terminal B15 in the medical institution B 1 transmits the request for displaying the divergence information via the I/F unit 105X to the integrated data management device A. At this time, the controller 100X functions as the divergence information display request section X5 shown in FIG. 13.

In step ST41 in FIG. 19, the integrated data management device A receives the display request from the expert meeting terminal B15 in the medical institution B 1. Subsequently, the integrated data management device A generates the divergence information in step ST42 in FIG. 19. At this time, the controller 100A functions as the divergence information generation section A19 shown in FIG. 6. The process of generating the divergence information will be described below. The generated divergence information is transmitted to the expert meeting terminal B15.

The expert meeting terminal B15 in the medical institution B 1 displays the divergence information transmitted from the integrated data management device A, on the output unit 107X such as a display, via the browser software, in step ST82. At this time, the controller 100X functions as the divergence information output section X7 shown in FIG. 13.

The expert meeting terminal B 15 in the medical institution B1 receives a mail of the expert meeting schedule transmitted from the integrated data management device A, via the mail software in the expert meeting terminal B15 in the medical institution B1. At this time, the controller 100X functions as the schedule reception section X3 shown in FIG. 13.

Subsequently, the expert meeting terminal B15 in the medical institution B 1 transmits, to the integrated data management device A, a request for a reservation for the expert meeting for the patient of the doctor H1a in charge, in step ST85. The integrated data management device A updates the schedule based on the received request for the reservation, in step ST45. The processes in step ST45 and ST85 will be described below.

Subsequently, the integrated data management device A outputs the updated expert meeting schedule in step ST46. The output is, for example, implemented by transmission of the expert meeting schedule to participants of the expert meeting with use of the mail software. At this time, the controller 100A functions as the schedule output section A21 shown in FIG. 6.

Next, the flow of the process performed between the integrated data management device A and the bureau expert meeting terminal SP15, G35 will be described. The process performed by the expert meeting terminal SP15 and the process performed by the bureau expert meeting terminal G35 are the same. Therefore, the flow of the process performed by the bureau expert meeting terminal G35 will be described.

A bureau staff member (hereinafter, simply referred to also as bureau staff member) of the expert meeting accesses the integrated data management device A via the browser software from the bureau expert meeting terminal G35. In step ST101 in FIG. 19, the controller 100Z (hereinafter, simply referred to as "bureau expert meeting terminal G35") of the bureau expert meeting terminal G35 receives a request for displaying the divergence information, through the input unit 106Z, from the bureau staff member. Subsequently, the bureau expert meeting terminal G35 transmits the request for displaying the divergence information, via the I/F unit 105Z, to the integrated data management device A. At this time, the controller 100Z functions as the divergence information display request section Z3 shown in FIG. 15.

The integrated data management device A receives the display request from the bureau expert meeting terminal G35 in the medical institution G in step ST41 shown in FIG. 19. Subsequently, the integrated data management device A generates the divergence information in step ST42 shown in FIG. 19. At this time, the controller 100A functions as the divergence information generation section A19 shown in FIG. 6. The process of generating the divergence information will be described below.

The bureau expert meeting terminal G35 displays the divergence information outputted by the integrated data management device A, on the output unit 107Z such as a display, via the browser software, in step ST102. At this time, the controller 100Z functions as the divergence information output section Z5 shown in FIG. 15.

The bureau staff member of the expert meeting adds a scheduled date for holding the expert meeting or adds meeting frames on the scheduled meeting date through the bureau expert meeting terminal G35 as necessary. The bureau expert meeting terminal G35 receives setting of the meeting schedule through the input unit 106Z shown in FIG. 12, in step ST103. At this time, the controller 100Z functions as the schedule setting section Z2 shown in FIG. 15. Adding a scheduled date for holding the expert meeting and adding a meeting frame will be described below in detail.

The integrated data management device A performs a schedule updating process in ST45. In the schedule updating process, the master table M and the expert meeting schedule database SDB are updated based on the set number of the meetings and the scheduled date, for holding the expert meeting, having been transmitted from the bureau expert meeting terminal G35 in step ST103, and the reservation request information transmitted in step ST85. At this time, the controller 100A functions as the schedule updating section A20 shown in FIG. 6.

Subsequently, the integrated data management device A outputs the set expert meeting schedule in step ST46 in FIG. 19. The output is, for example, implemented by transmission of the expert meeting schedule to the participants of the expert meeting with use of mail software. At this time, the controller 100A functions as the schedule output section A21 shown in FIG. 6.

The bureau expert meeting terminal G35 receives the mail of the expert meeting schedule transmitted from the integrated data management device A by using mail software in the bureau expert meeting terminal G35 in step ST104 shown in FIG. 19. At this time, the controller 100Z functions as the schedule reception section Z1 shown in FIG. 15.

8-4. Process of generating divergence information

The process performed by the integrated data management device A as described below is configured as a part or the entirety of the computer program for supporting, by using a computer, management of the expert meeting in which a plurality of medical workers interpret gene information of a patient.

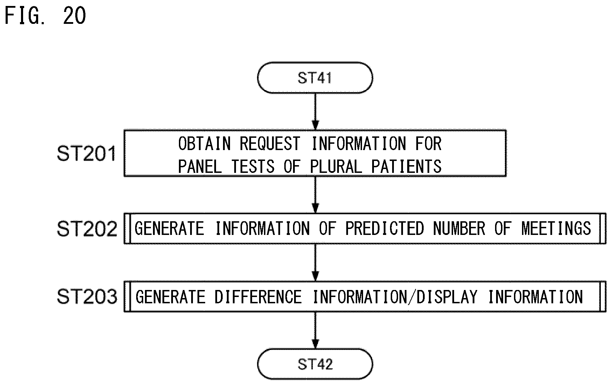

The integrated data management device A performs the divergence information generation process shown in FIG. 20 by using the program for generating the divergence information. Unless otherwise specified, the process is performed by the controller 100A.

The integrated data management device A is triggered by receiving the display request in step ST41 in FIG. 19 to start the divergence information generation process. Alternatively, the integrated data management device A may generate the divergence information periodically, for example, every hour or every day.

### (1) Flow of entire process

In step ST201 in FIG. 20, the integrated data management device A obtains the request information, for a plurality of panel tests, stored in the master table M for a plurality of patients.

In step ST202, the integrated data management device A generates information of the predicted number of the meetings, based on the panel test request information obtained in step ST201.

Subsequently, in step ST203, the integrated data management device A generates the divergence information based on the information of the predicted number of the meetings generated in step ST202. The divergence information is not particularly limited as long as the information indicates a difference between the set meeting time frames and the predicted number of the meetings, such that the difference is recognizable.

### (2) Step of generating information of predicted number of meetings

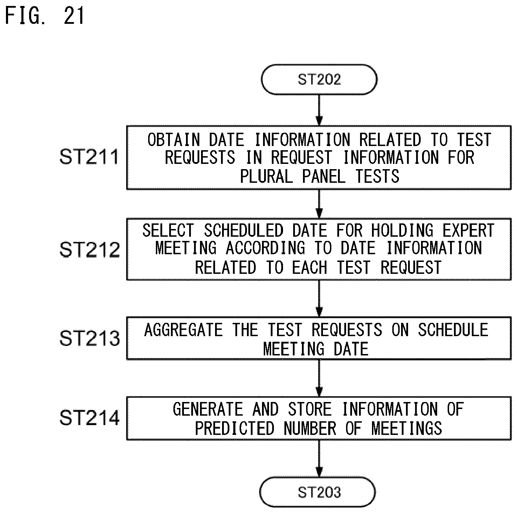

The process in step ST202 for generating the information of the predicted number of the meetings will be described in detail with reference to FIG. 21.

In step ST211 in FIG. 21, the integrated data management device A obtains date information related to the test request, from among the request information of each panel test having been obtained in step ST201 in FIG. 20. The date information related to the test request may be any of the test request date, the test reception date, and the specimen reception date. In the example described herein, the test request date is used.

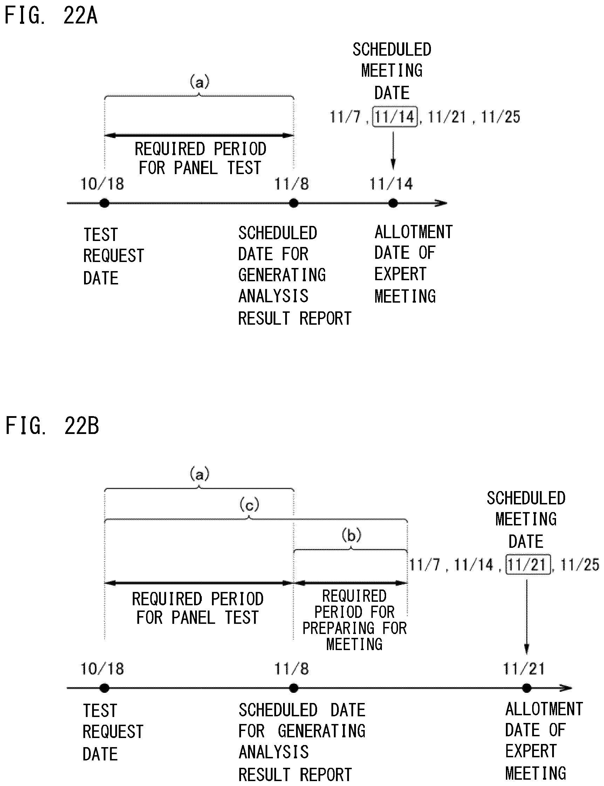

Next, in step ST212, the integrated data management device A selects the scheduled date for holding the expert meeting, i.e., the allotment date from among a plurality of scheduled dates, for holding the expert meetings, which are previously stored in the expert meeting schedule database SDB, for each gene panel test. The method for selecting the allotment date will be described with reference to FIG. 22. In the description with reference to FIG. 22A, the test request date (October 18) is the start date for the counting. For example, a period from the test request to generation of the analysis report is about three weeks in the gene panel test. In FIG. 22A, the period is indicated as the required days (a). The scheduled date for holding the expert meeting needs to be selected in consideration of the required days (a). The scheduled meeting date can be set as the earliest available scheduled meeting date (November 14) after the required period (a) has elapsed from the test request date among the plurality of scheduled dates, for holding the expert meetings, which are stored in the expert meeting schedule database SDB. The selected scheduled meeting date is stored in the "allotment date" region in the master table M so as to be associated with the request information of the panel test of each patient.

The required period (a) may include a preparation period from generation of the analysis report until holding of the expert meeting. In FIG. 22B, a required period (c) is set by adding a preparation period (b) from generation of the analysis report until holding of the expert meeting, to the required period (a) from the test request date to the generation of the analysis report.

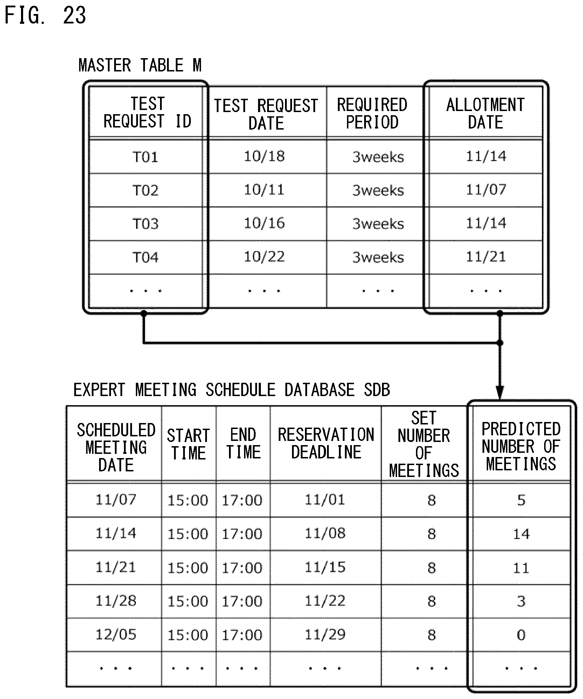

Returning to FIG. 21, in step ST213, the integrated data management device A aggregates the test requests for each of the plurality of scheduled meeting dates stored in the expert meeting schedule database SDB. FIG. 23 shows the example of the aggregation. Each allotment date stored in the master table M is associated with the test request ID in the master table M. The integrated data management device A counts the number of the test requests according to the test request IDs associated with the same allotment date, and sets the counted number as the number of meetings on the scheduled meeting date.

For example, in the example in FIG. 23, the number of the test request IDs for the allotment date of November 7 is five including the test request ID=T02 (cells other than the cells for T02 are not shown), the number of the test request IDs for the allotment date of November 14 is 14 including the test request ID=T01 and T03 (cells other than the cells for T01 and T03 are not shown), and the number of the test request IDs for the allotment date of November 21 is 11 including the test request ID=T04 (cells other than the cells for T04 are not shown).

The test requests may be aggregated by counting the patient IDs as the number of the test requests.

Returning to FIG. 21, the integrated data management device A generates the information of the predicted number of the meetings by setting the aggregate number of the test requests as the predicted number of frames for the meeting in step ST214. The scheduled meeting date and the information of the predicted number of the meetings are stored, by the integrated data management device A, in the expert meeting schedule database SDB in the comprehensive database OG so as to be associated with each other, as shown in FIG. 23.

### (3) Step of generating divergence information

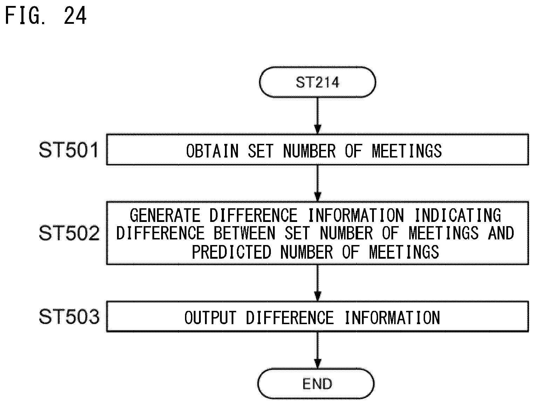

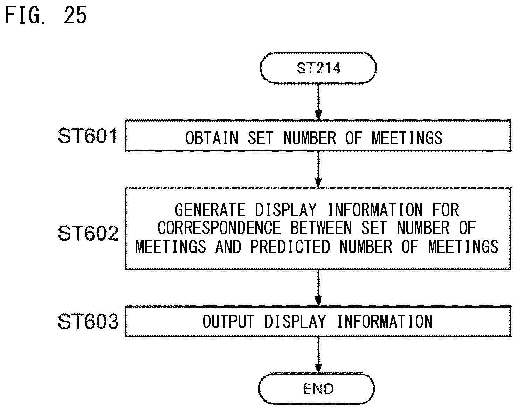

Next, a method for generating the divergence information will be described with reference to FIG. 24 and FIG. 25. The divergence information includes at least one selected from difference information indicating a difference between the set number of meetings and the predicted number of the meetings generated in step ST214 in FIG. 21, and display information for displaying the set number of meetings and the predicted number of the meetings generated in step ST214 in FIG. 21 so as to associate the set number of meetings and the predicted number of the meetings with each other.

### (i) Example 1 of generating divergence information

A specific process in a difference information generation step will be described as an example of generating the divergence information with reference to FIG. 24.

The integrated data management device A obtains information of the set number of meetings, from the expert meeting schedule database SDB stored in the comprehensive database OG, in step ST501. This step is performed continuously after step ST214 in FIG. 21.

Next, in step ST502, the integrated data management device A generates the difference information based on the information of the predicted number of the meetings generated in step ST214 in FIG. 21 and the information of the set number of meetings obtained in step ST501 in FIG. 24. Specifically, the difference information is information that includes a subtraction value obtained by subtracting the predicted number of the meetings from the set number of meetings. The information including the subtraction value may be, for example, the subtraction value itself, a label indicating that the subtraction value is a positive value, or a label indicating that the subtraction value is a negative value. The label indicating that the subtraction value is a positive value indicates that the number of meeting frames is sufficient for the scheduled meeting date. The label indicating that the subtraction value is a negative value indicates that the number of meeting frames is insufficient for the scheduled meeting date.

The integrated data management device A outputs the generated difference information in step ST503.

### (ii) Example 2 of generating divergence information

A specific process in a display information generation step will be described as another example of generating the divergence information with reference to FIG. 25.

The integrated data management device A obtains information of the set number of meetings, from the expert meeting schedule database SDB stored in the comprehensive database OG, in step ST601. This step is performed continuously after step ST214 in FIG. 21.

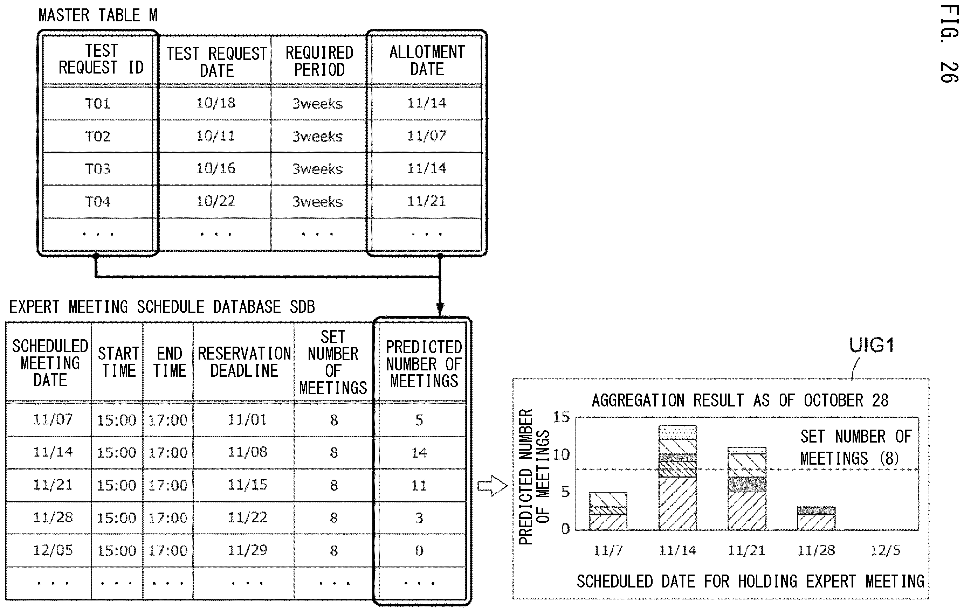

Next, in step ST602, the integrated data management device A generates display information based on the information of the predicted number of the meetings generated in step ST214 in FIG. 21, and the information of the set number of meetings obtained in step ST601 in FIG. 25. The display information is not particularly limited as long as the information of the set number of meetings and the information of the predicted number of the meetings can be displayed so as to be associated with each other. FIG. 26 shows a graphical interface UIG1 as an example of the display information. In the example in FIG. 26, the information of the predicted number of the meetings is displayed as the predicted number of meeting frames in the form of a bar chart for each of the scheduled dates for holding the expert meetings, in the graphical interface UIG1. The number of cases, for each hospital, covered by one expert meeting is indicated by difference in hatched patterns in the graph. In FIG. 26, the information of the set number of meetings is indicated by a dashed line. The dashed line is indicated at a position corresponding to the set number of meetings. In the example in FIG. 26, the set number of meetings is indicated as eight. The bar in which the height of the line is above the dashed line in the bar chart indicates that the number of meeting frames is insufficient for the scheduled meeting date (November 14 and November 21 in FIG. 26). The bar in which the height of the line is not above the dashed line in the bar chart indicates that the number of meeting frames is sufficient for the scheduled meeting date (November 7, November 28, and December 5 in FIG. 26).

The integrated data management device A outputs the generated display information in step ST603.

### 8-5. Process of adding scheduled meeting date

In a case where the number of meeting frames is insufficient, the bureau staff member adds a scheduled date for holding the expert meeting from the bureau expert meeting terminal G35 as necessary in step ST103 in FIG. 19 as described above.

For example, this process is performed by a process in which the integrated data management device A is accessed through the bureau expert meeting terminal SP15, G35, and a new scheduled meeting date is registered or a scheduled meeting date is changed and registered in the expert meeting schedule database SDB stored in the comprehensive database OG of the integrated data management device A.

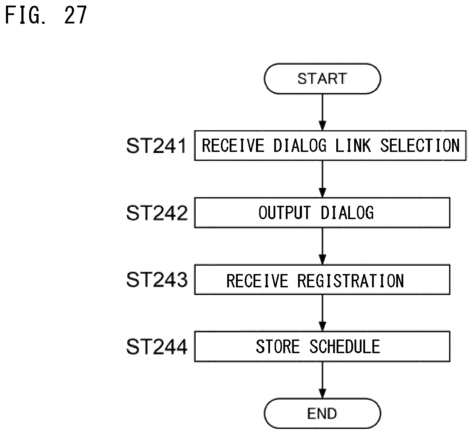

The process of adding a scheduled date for holding the expert meeting will be described with reference to FIG. 27 to FIG. 29. The process performed by the expert meeting terminal SP15 and the process performed by the bureau expert meeting terminal G35 are the same. Therefore, the flow of the process will be described by using the bureau expert meeting terminal G35.

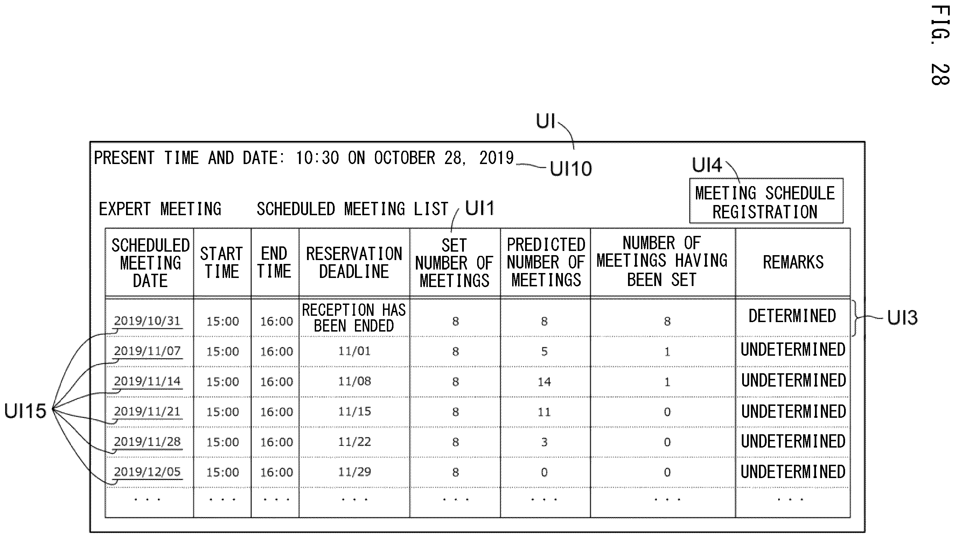

In step ST241 in FIG. 27, the integrated data management device A receives, through the bureau expert meeting terminal G35, an instruction for selecting a link to a dialog for setting of a meeting inputted by the bureau staff member from the bureau expert meeting terminal G35. FIG. 28 shows an example of link information for linking to the dialog for setting of a meeting. FIG. 28 shows an example of a graphical user interface UI, for displaying a list of scheduled expert meetings to be held, which is linked to the information stored in the expert meeting schedule database SDB. The graphical user interface UI includes a region UI10 indicating a time at which the list of scheduled expert meetings to be held is accessed. The graphical user interface UI includes a scheduled meeting list region UI1 which is stored in the expert meeting schedule database SDB and includes cells for indicating remarks and the number of frames having been set, in addition to a scheduled meeting date, a start time, an end time, a reservation deadline, the set number of meetings, and the predicted number of frames. The scheduled meeting list region UI1 includes a specific scheduled meeting date display region UI3 for indicating information for each of the scheduled meeting dates. The scheduled meeting date cell in each line in the scheduled meeting list region UI1 includes link information UI15 that allows access to a dialog for registering a scheduled meeting date. Furthermore, the graphical user interface UI includes a scheduled meeting registration icon UI4 having link information that allows access to a dialogue for registering a scheduled meeting date.

In a case where the bureau staff member newly registers a scheduled date for holding the expert meeting, when the integrated data management device A receives an instruction for selecting the scheduled meeting registration icon UI4 in step ST241 in FIG. 27, the process proceeds to step ST242, to display a dialogue D4 for inputting a scheduled meeting date.

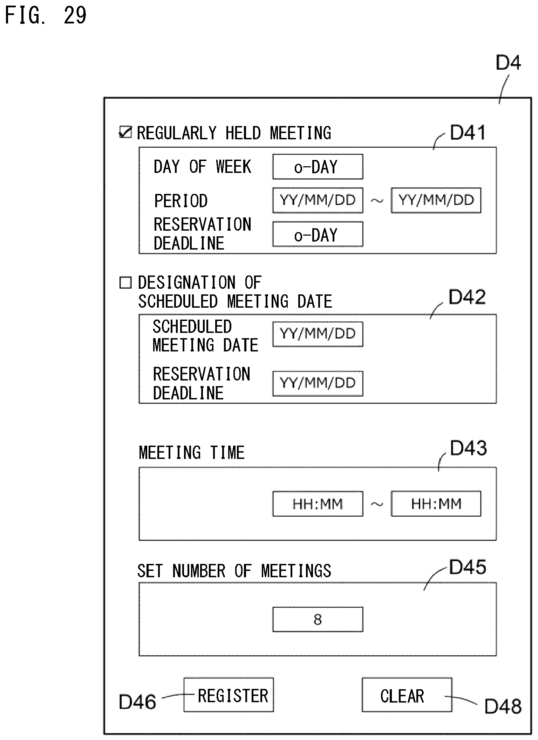

FIG. 29 shows an example of the dialogue D4. The dialogue D4 includes a region D41 for inputting a scheduled date for regularly held meetings, a region D42 for designating and inputting a scheduled meeting date, a region D43 for designating a time of the meeting to be held, a region D45 for registering the set number of meetings, a registration icon D46 for determining the setting, and a clear icon D48 for clearing the input. The region D41 may include a region for allowing designation of a day of week, a region for designating a meeting period, and a region for designating a reservation deadline. The region D42 may include a region for designating a scheduled meeting date and a region for designating a reservation deadline.

In step ST243 in FIG. 27, when the bureau staff member inputs through the input unit 106Z information related to the scheduled meeting date such as a scheduled meeting date, a scheduled deadline, a meeting time, and the set number of meetings, which are to be added in the regions D42, D43, D45, and selects the registration icon D46, the integrated data management device A receives registration of the new scheduled meeting date. Subsequently, the integrated data management device A stores information related to the inputted new scheduled meeting date in the expert meeting schedule database SDB in step ST243, to update the expert meeting schedule database SDB. The processes in step ST241 to step ST243 correspond to the process in step ST45 performed based on the information transmitted from the bureau expert meeting terminal G35 in the process in step ST103 shown in FIG. 19. The updated contents in the expert meeting schedule database SDB are outputted as regenerated divergence information in step S46 shown in FIG. 19.

In a case where the scheduled meeting date registered by the bureau staff member is updated, when the integrated data management device A receives, in step ST241 in FIG. 27, an instruction for selecting the link information UI15 corresponding to a date for which update is to be performed, the process proceeds to step ST242, to display the dialogue D4 for inputting the scheduled meeting date. In this case, the dialogue D4 is displayed such that the date for which the update is to be performed is displayed in the region D42, a check box next to "regular meeting" is not checked, and a check box for "designated scheduled meeting date" is checked. The processes in step S243 and step S244 are the same as the processes for newly registering a scheduled meeting date.

### 8-5. Process of changing scheduled meeting date or scheduled meeting time

In a case where the number of meeting frames is insufficient, the bureau staff member adds a scheduled date for holding the expert meeting and/or adds the number of meeting frames from the bureau expert meeting terminal G35 as necessary in step ST103 shown in FIG. 19, as described above.

This process is performed by, for example, accessing the integrated data management device A through the bureau expert meeting terminal SP15, G35, and additionally registering a scheduled meeting date and updating the set number of frames in the expert meeting schedule database SDB stored in the comprehensive database OG of the integrated data management device A.

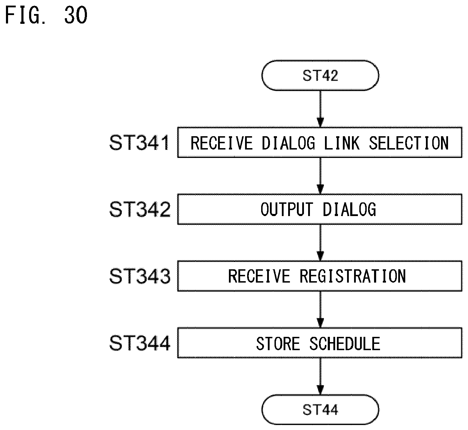

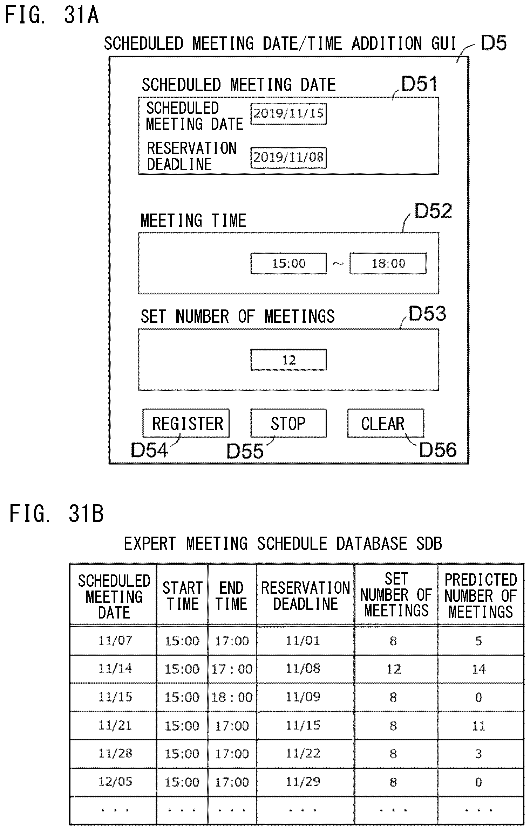

The process of adding a scheduled date for holding the expert meeting and adding the set number of frames will be described with reference to FIG. 30 and FIG. 31. The process performed by the expert meeting terminal SP15 and the process performed by the bureau expert meeting terminal G35 are the same. Therefore, the flow of the process will be described by using the bureau expert meeting terminal G35.

In step ST341 in FIG. 30, the integrated data management device A receives an instruction for selecting a link to a dialogue for setting of a meeting inputted by the bureau staff member from the bureau expert meeting terminal G35. An example of the link to the dialogue for setting of a meeting is the same as shown in FIG. 28.

When the integrated data management device A receives, in step ST341 in FIG. 30, the instruction for selecting the scheduled meeting registration icon UI4 or the link information UI15 in the scheduled meeting date cell in which the bureau staff member has made an input from the bureau expert meeting terminal G35, the process proceeds to step ST342, to display a dialogue D5 for inputting an additional scheduled meeting date.

FIG. 31A shows an example of the dialogue D5. The dialogue D5 includes a region D51 for inputting an additional scheduled meeting date, a region D52 for designating a meeting time, a region D53 for registering the set number of meetings, a registration icon D54 for determining the setting, a stop icon D55 for stopping the setting, and a clear icon D56 for clearing the input. The region D51 may include a region for designating a scheduled meeting date and a region for designating a reservation deadline. In a case where the instruction for selecting the link information UI15 is received in step ST341, the scheduled meeting date corresponding to the selected link information UI15 is previously displayed in the region D51. In a case where the instruction for selecting the scheduled meeting registration icon UI4 is received in step ST341, no scheduled meeting date is displayed in the region D51.

In step ST343 shown in FIG. 30, when the bureau staff member changes the scheduled meeting date in the region 51, the scheduled meeting time in the region 52, and/or the set number of meetings in the region 53 as shown in FIG. 31A, through the input unit 106Z, and selects the registration icon D54, the integrated data management device A receives registration of addition of the scheduled meeting date, addition of the reservation deadline, change of the scheduled meeting time, and change of the set number of meetings. Subsequently, in step ST344, the integrated data management device A stores information related to the scheduled meeting date, the reservation deadline, the scheduled meeting time, and the set number of meetings, which have been inputted, in the "scheduled meeting date", "start time", "end time", "reservation deadline", and "set number of meetings" regions in the expert meeting schedule database SDB, to update the expert meeting schedule database SDB. In the example shown in FIG. 31A, November 15, 2019 is added as the scheduled meeting date, November 8, 2019 is inputted as the reservation deadline, the meeting time on that day is inputted as 15:00 to 18:00, and 12 is inputted as the set number of meetings, so as to be reflected in the expert meeting schedule database SDB shown in FIG. 31B. The processes in step ST341 to step ST344 correspond to the process in step ST45 performed based on the information transmitted from the bureau expert meeting terminal G35 in the process in step ST103 shown in FIG. 19. The contents updated in the expert meeting schedule database SDB are outputted as the regenerated divergence information in step ST46 in FIG. 19.

### 8-6. Modifications

### (1) Example of display

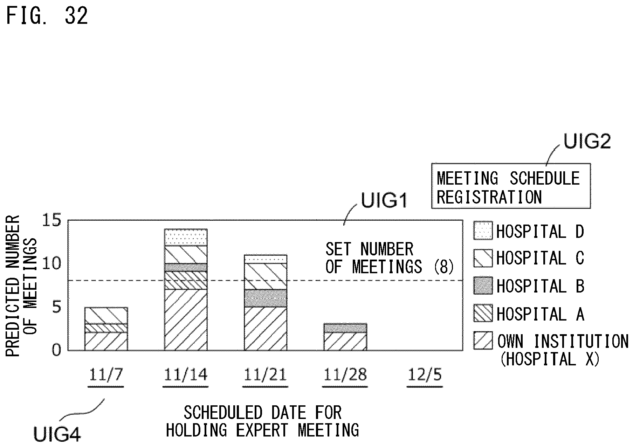

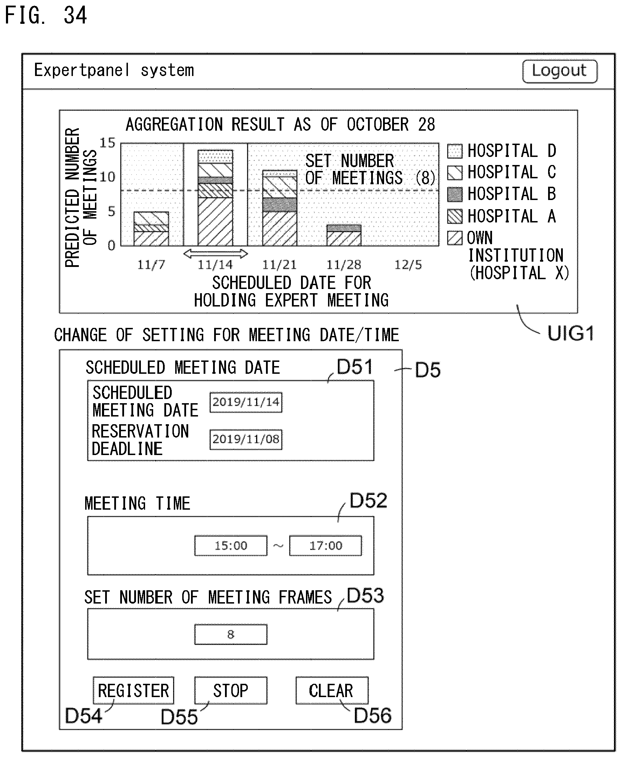

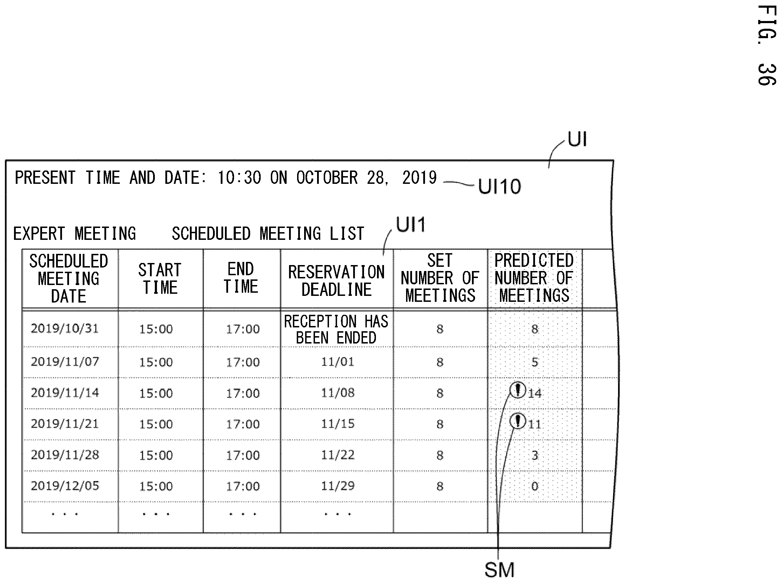

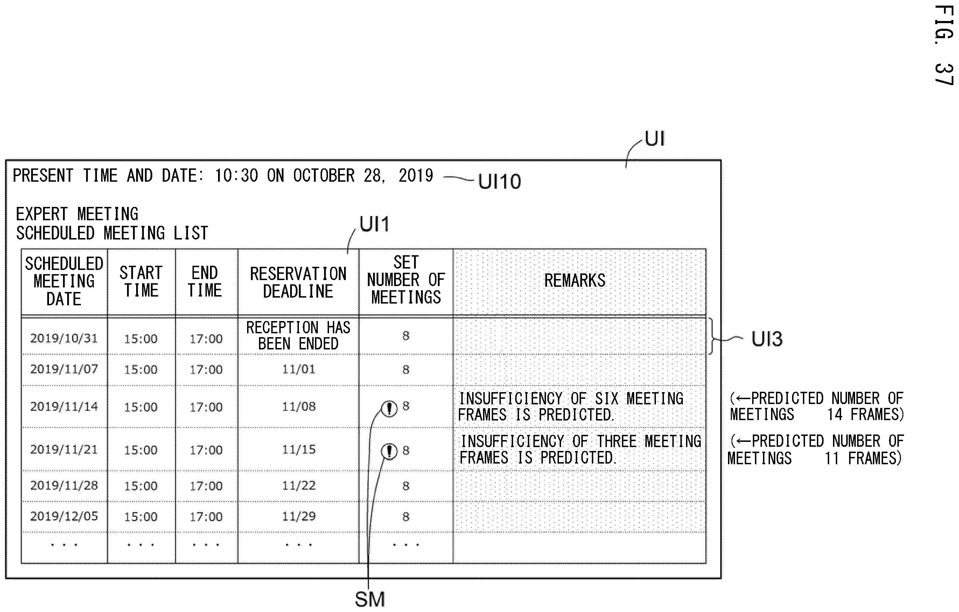

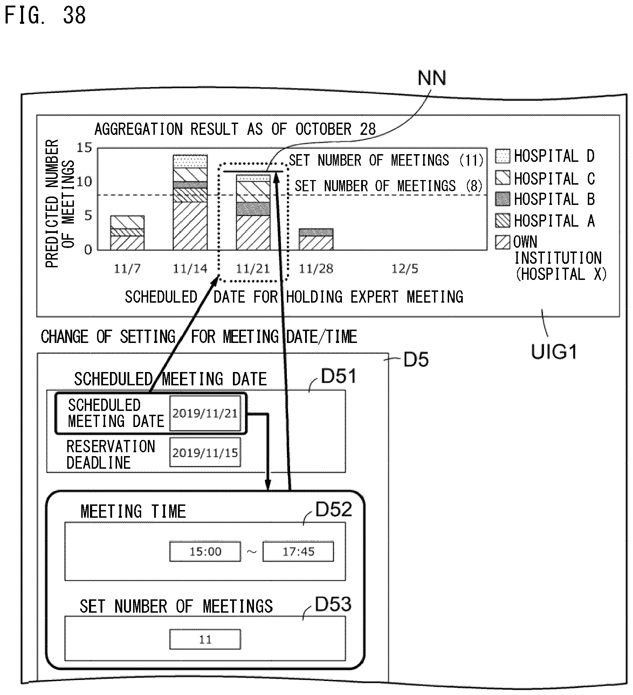

(i) FIG. 32 shows another example of a graphical user interface including link information that allows access to a dialogue for newly registering a scheduled meeting date. FIG. 32 shows an example in which the graphical interface UIG1 generated in step ST603 shown in FIG. 25 includes a scheduled meeting registration icon UIG2 having link information that allows access to a dialogue for registering a scheduled meeting date. Furthermore, the link information that allows access to a dialogue for updating a meeting time and the set number of meetings for the scheduled meeting date may be set at a character string UIG4 indicating the scheduled meeting date for the meeting.

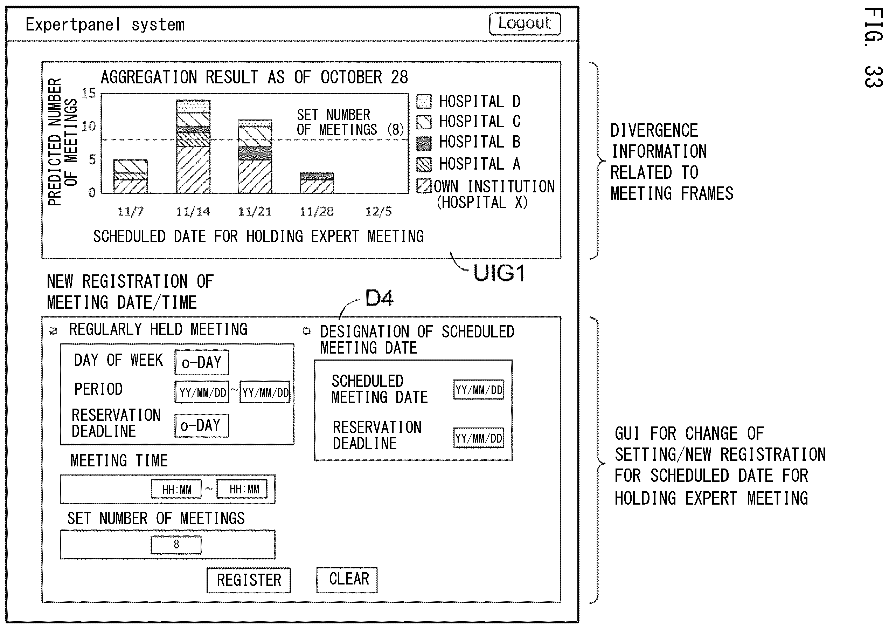

(ii) FIG. 33 shows another example of display of the dialogue D4 for inputting a scheduled meeting date. FIG. 33 shows an example in which the graphical interface UIG1 generated in step ST603 in FIG. 25, and the dialogue D4 shown in FIG. 29 are displayed on one screen. The elements in FIG. 33 correspond to the elements in the graphical interface UIG1 and the elements in the dialogue D4. The graphical interface UIG1 may be displayed such that, in a case where the scheduled meeting date is added through the dialogue D4, the addition is also displayed in the graphical interface UIG1. Furthermore, in FIG. 33, the dialogue D4 may be replaced by the dialogue D5.
(iii) FIG. 34 shows a modification of the example of the display shown in FIG. 33. In FIG. 34, the dialogue D4 in FIG. 33 is changed to the dialogue D5. Furthermore, when the scheduled meeting date, the scheduled meeting time, or the set number of meetings is changed in the dialogue D5, the scheduled meeting date for which change is made and the scheduled meeting date for which no change is made may be displayed so as to be distinguished from each other in the graphical interface UIG1. For example, in the example in FIG. 34, hatching is made for the scheduled meeting dates (November 7, November 21, November 28, and December 5) for which no change is made.

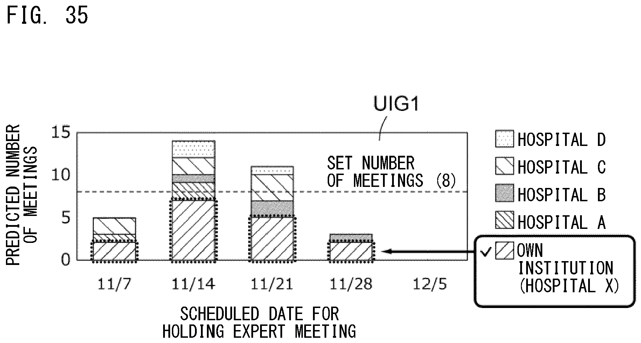

(iv) FIG. 35 shows an example in which, when a name of an institution in the list is clicked or pointed on the graphical interface UIG1, the clicked or pointed institution is displayed so as to be distinguished from the other institutions in the bar chart. In FIG. 35, portions corresponding to the clicked or pointed institution (hospital X) that is selected in the bar chart is surrounded by dashed lines and displayed. The display method is not limited to that using the surrounding lines, and may be, for example, a method in which color is changed or emphasis is made by hatching, or a three-dimensional display.
(v) In the above-described embodiments, the display information is represented by the bar chart. However, the display format is not limited to that using a bar chart.
(vi) The display information may be displayed in the graphical user interface UI for displaying the scheduled expert meeting list as shown in, for example, FIG. 36 and FIG. 37. In the example shown in FIG. 36, the predicted number of the meetings (the predicted number of the meetings on the scheduled meeting date of November 14: 14, and the predicted number of the meetings on the scheduled meeting date of November 21: 11) that is greater than the set number of meetings is indicated with an exclamation mark SM. In order to indicate that the predicted number of the meetings is greater than the set number of meetings, as shown in FIG. 37, a message indicating that insufficiency of the meeting frames is predicted may be displayed.
(vii) FIG. 38 shows a modification of the example of the display shown in FIG. 33. In FIG. 38, the dialogue D4 in FIG. 33 is changed to the dialogue D5. Furthermore, in the dialogue D5, when a scheduled meeting date is inputted in the region D51, the integrated data management device A may specify the predicted number of frames for the corresponding scheduled meeting date in the expert meeting schedule database SDB, and the meeting time in the region D52 and/or the set number of meetings in the region D53 may be automatically changed so as to meet the predicted number of frames. Moreover, the display in the graph may be changed according to the changed set number of meetings. For example, in the example shown in FIG. 38, November 21 is inputted as the scheduled meeting date in the region D51, and the meeting time in the region D52 is changed to 15:00 to 17:45 and the set number of meetings in the region D53 is changed to 11 so as to meet the predicted number of the meetings (11 in this example) for the date. In the example shown in FIG. 38, display of the set number of meetings on November 21 in the graph is changed from eight to eleven (the changed set number of meetings is displayed as a horizontal line NN).

### 8-7. Process of making reservation for meeting from expert meeting terminal in medical institution

Next, a process of making a reservation for the meeting from the expert meeting terminal in the medical institution will be described. This process corresponds to the processes in step ST45 and ST85 in FIG. 19, and is performed when the doctor H1a in charge makes a reservation for the expert meeting.

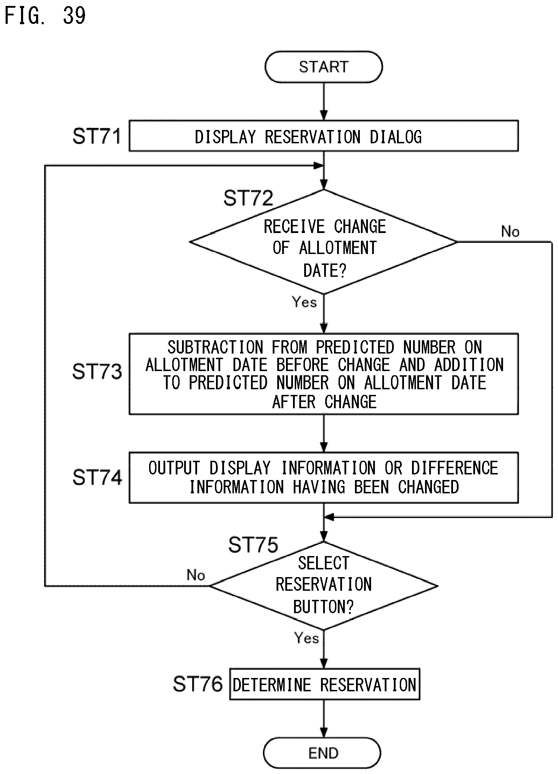

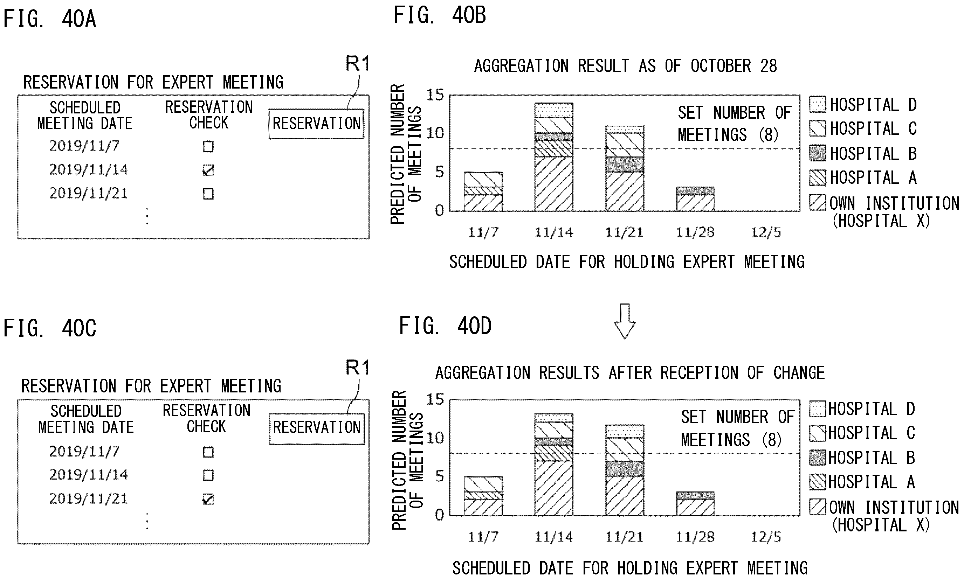

The meeting reservation process will be described with reference to FIG. 39 and FIG. 40.

In step ST71 in FIG. 39, the integrated data management device A displays a reservation dialogue for making a reservation for an expert meeting for a patient of the doctor H1a in charge, on the expert meeting terminal B15 in the medical institution. FIG. 40A and FIG. 40C each show an example of the reservation dialogue. A plurality of scheduled meeting dates stored in the "scheduled meeting date" region in the expert meeting schedule database SDB, check boxes corresponding to the respective scheduled meeting dates, and a reservation button R1 for determining the reservation are displayed in the reservation dialogue. Furthermore, in the example shown in FIG. 40A, the scheduled meeting date of November 14 is checked. The scheduled meeting date of November 14 is the scheduled meeting date selected in the process in step S212 shown in FIG. 21, that is, the allotment date.

In step ST72, the integrated data management device A determines whether or not change of the allotment date has been received. In a case where the position of the check is changed as shown in, for example, FIG. 40C in the reservation dialogue displayed on the expert meeting terminal B15 in the medical institution, the information thereof is transmitted from the expert meeting terminal B 15 in the medical institution to the integrated data management device A, and the integrated data management device A determines that the change of the allotment date has been received.

In a case where the change of the allotment date has been received (Yes in step ST72), the integrated data management device A subtracts 1 from the predicted number of the meetings on the scheduled meeting date (November 14 in the example shown in FIG. 40) corresponding to the allotment date before the change in the expert meeting schedule database SDB, and adds 1 to the predicted number of the meetings on the scheduled meeting date (November 21 in the example shown in FIG. 40) corresponding to the allotment date after the change, in step ST73.

In a case where change of the allotment date is not received (No in step ST72), the integrated data management device A advances the process to step ST75.

Next, in step ST74, the integrated data management device A generates the difference information or the display information according to step ST501 to step ST502 shown in FIG. 24 or step ST601 to step ST602 shown in FIG. 25. FIG. 40B shows an example of unchanged display information. FIG. 40D shows an example of changed display information.

Next, the integrated data management device A determines in step ST75 whether or not the reservation button R1 has been selected in the reservation dialogue. In a case where the reservation button R1 has been selected (Yes in step ST75), the process proceeds to step ST76, and the reservation flag in the master database M is changed from 0 to 1, thereby determining the reservation. In a case where the reservation button R1 is not selected (No in step ST75), the integrated data management device A returns the process to step ST72.

### 8-8. Setting of set number of meetings according to modification

In the embodiments described above in 8-1. to 8-7., the set number of meetings is based on the set number of meetings according to the preset frames (also referred to as default value). Furthermore, the flow of the process has been described based on a case where the bureau staff member changes the set number of meetings.

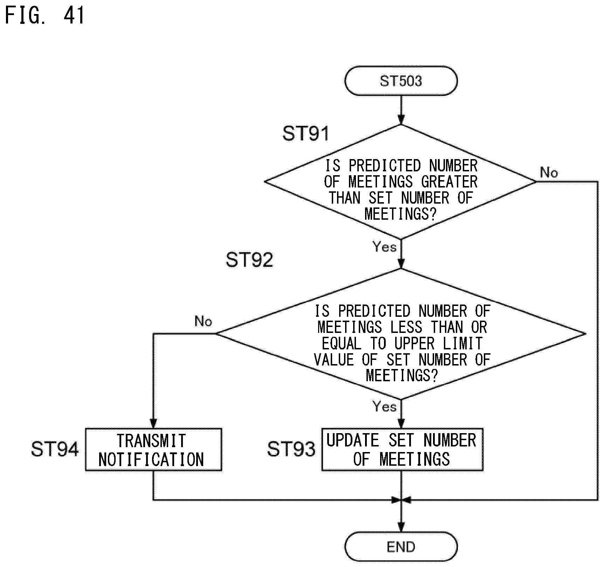

In this modification, an example in which the integrated data management device A automatically changes the set number of meetings will be described with reference to FIG. 41. For example, the set number of meetings is changed based on the difference information generated in step ST502 in FIG. 24.

The integrated data management device A determines in step ST91 whether or not the predicted number of the meetings is greater than the set number of meetings. In a case where the predicted number of the meetings is not greater than the set number of meetings (in the case of "No"), the process is ended. In a case where the predicted number of the meetings is greater than the set number of meetings (in the case of "Yes"), the integrated data management device A advances the process to step ST92.

The integrated data management device A determines in step ST92 whether or not the predicted number of the meetings is greater than the upper limit value of the set number of meetings. The upper limit value of the set number of meetings is preset for each of the institutions at which the expert meeting is held, and stored in the comprehensive database OG. The upper limit value of the set number of meetings is the number obtained by adding a predetermined number to the set number of meetings, and may be, for example, the maximum number of the meetings that can be held on the scheduled date for holding the expert meeting. In a case where the predicted number of the meetings is less than or equal to the upper limit value of the set number of meetings (in the case of "Yes"), the integrated data management device A advances the process to step ST93, and changes the set number of meetings in the expert meeting schedule database SDB to the number that can meet the predicted number of the meetings, to end the process. In a case where the predicted number of the meetings is greater than the upper limit value of the set number of meetings (in the case of "No"), the integrated data management device A transmits a mail or the like for notifying the bureau expert meeting terminal G35 that the predicted number of the meetings is greater than the upper limit value, in step ST94. The processes in step ST91 to step ST94 are performed for each of the scheduled meeting dates.

Thus, in a case where the predicted number of the meetings is merely greater than the set number of meetings, the bureau expert meeting terminal G35 is not notified thereof, and the set number of meetings is updated. In a case where the predicted number of the meetings is greater than the upper limit value of the set number of meetings, the bureau expert meeting terminal G35 is notified thereof. Thus, the number of meetings can be appropriately set while reducing work load on the bureau of the expert meeting.

Instead of the processes in step ST91 to step ST94, the integrated data management device A may set the set number of meetings, for example, according to the number of expert meetings that were previously held. More specifically, for example, an average value of the meeting frames per one day in the corresponding institution within the past six months or the past one year is calculated, and the average value may be set as the set number of meetings.

### 8-9. Method for setting allotment date according to modification

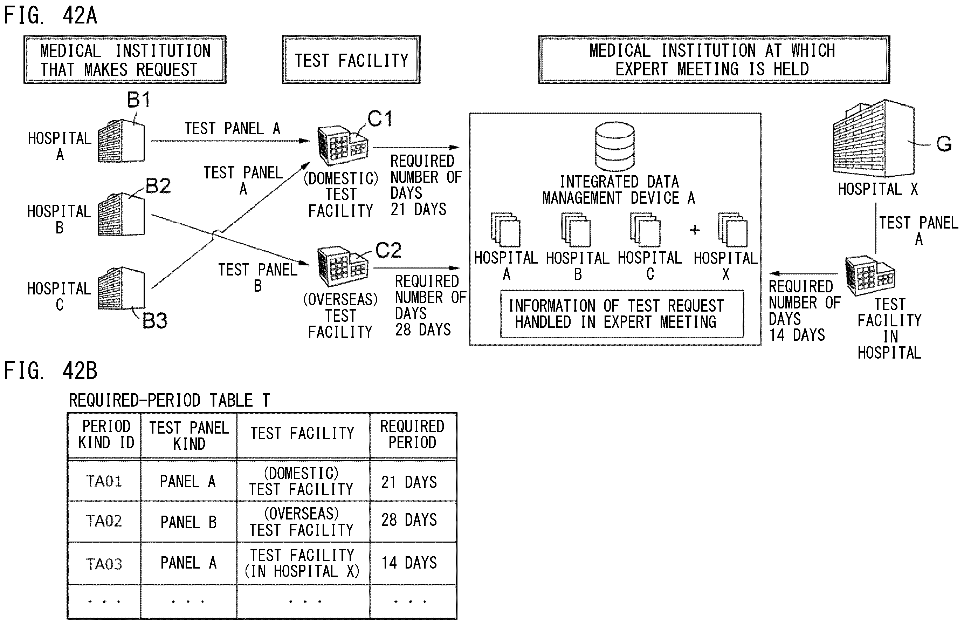

Another example of the method for setting the allotment date shown in FIG. 22 will be described with reference to FIG. 42.

As shown in FIG. 42A, some of the gene panel tests are performed at the domestic test facility C1, and the other of the gene panel tests are performed at the overseas test facility C2. Furthermore, also in a case where the gene panel test is domestically performed, the required period (required period (a) in FIG. 22A) from the test request date, the test request reception date, or the specimen reception date to generation of the analysis report may be different according to whether the test is committed to an external facility or performed in the institution consulted by a patient. For example, according to setting in a required-period table T shown in FIG. 42B, the required period is 21 days in a case where a test for a panel A is performed at a domestic test facility, the required period is 14 days in a case where the test for the panel A is performed at the hospital X, and the required period is 28 days in a case where a test for a panel B is performed at an overseas test facility. The required-period table T is stored in the comprehensive database OG of the integrated data management device A. The integrated data management device A extracts the required period stored in the required-period table T, for each specimen ID, by using a test facility and a gene panel ID as keys, and stores the required period in the "required period" region in the master table M. Thus, in step S212 in FIG. 21, when the scheduled date for holding the expert meeting is selected, the scheduled meeting date can be selected according to the test facility and/or a kind of the gene panel, and the divergence information can be more accurately generated.

The required period may be set according to, for example, a disease of a patient as well as the test facility and/or a kind of the gene panel. A long time is required for generating the analysis report depending on a disease. Therefore, the divergence information can be more accurately generated.

### 8-10. Modification of meeting frame

In the embodiments described above in 8-1. to 8-9., the predicted meeting frames and the set meeting frames are indicated as the predicted number of the meetings and the set number of the meetings, respectively. However, the predicted meeting frames and the set meeting frames may be each indicated as a time by setting a time for one meeting. For example, the predicted meeting frames and the set meeting frames may be a time obtained by multiplying a time for one meeting by the predicted number of the meetings, and a time obtained by multiplying a time for one meeting by the set number of meetings, respectively. The predicted number of the meetings and a time calculated from the predicted number of the meetings and a time for one meeting may be both used as the predicted meeting frames. Similarly, the set number of the meetings and a time calculated from the set number of the meetings and a time for one meeting may be both used as the set meeting frames.

### 8-11. Range of set meeting frames

The set meeting frames may be allowed to be changed within a predetermined number of frames or time. The predetermined number of frames or time is preset by the bureau of the expert meeting, and stored in the comprehensive database OG.

### 9. Storage medium having computer program stored therein

A computer program for performing the process of step ST1 and step ST2; a computer program for performing the process of step ST21 to step ST30; a program for performing the process of step ST61 to step ST65; a computer program for performing the process of step ST41 to step ST46, step ST201 to step ST203, step ST211 to step ST214, and step ST501 to step ST503; a computer program for performing the process of step ST41 to step ST45, step ST201 to step ST203, step ST211 to step ST214, and step ST601 to step ST603; a computer program for performing the process of step ST241 to step ST244; a computer program for performing the process of step ST341 to step ST344; a computer program for performing the process of step ST71 to step ST74; and a computer program for performing the process of step ST91 to step ST94 can be provided as a program product such as a storage medium. The computer program is stored in a storage medium such as a hard disk, a semiconductor memory device such as a flash memory, and an optical disc. A form in which the program is stored in the storage medium is not particularly limited as long as the form can allow the controller to read the program. The program is preferably stored in the storage medium in a non-volatile manner.

## Claims

1. A support method for supporting, by using a computer, management of an expert meeting in which a plurality of medical workers interpret a result of a panel test for a patient, the support method comprising:
obtaining request information of a plurality of panel tests for patients for each of whom the expert meeting is held;
generating information of predicted meeting frames that are related to a number of meetings and/or a meeting time and that are predicted on a scheduled date for holding the expert meeting, based on the obtained request information of the plurality of panel tests; and
generating divergence information indicating a difference between the predicted meeting frames and set meeting frames that are related to a number of meetings and/or a meeting time and that are preset on the scheduled date, such that the difference is recognizable.

2. The support method of claim 1, wherein the divergence information includes display information for displaying the set meeting frames and the predicted meeting frames so as to associate the set meeting frames and the predicted meeting frames with each other.

3. The support method of claim 1 or 2, wherein the divergence information includes difference information indicating the difference between the set meeting frames and the predicted meeting frames.

4. The support method of any one of claims 1 to 3, wherein
the request information of the panel tests includes date information related to test requests, and wherein
generating the information of the predicted meeting frames comprises generating the information of the predicted meeting frames based on the date information related to the test requests.

5. The support method of claim 4, wherein the generating the information of the predicted meeting frames comprises generating the information of the predicted meeting frames by selecting the scheduled date from among a plurality of the scheduled dates, based on the date information related to the test requests, for the request information of each of the plurality of the panel tests, and aggregating the test requests for each of the plurality of the scheduled dates.

6. The support method of claim 4 or 5, wherein the generating the information of the predicted meeting frames comprises generating the information of the predicted meeting frames based on the date information related to the test requests and required-period information of each panel test.

7. The support method of claim 6, wherein
the request information of each panel test further includes information related to at least one selected from a kind of a test panel, a test facility, an institution at which the expert meeting is held, and a medical institution that makes a request, and
the generating the information of the predicted meeting frames comprises generating the required-period information of the panel test based on the information included in the request information.

8. The support method of any one of claims 4 to 7, wherein the date information includes at least one selected from a request date of each panel test, a reception date of the panel test, and a reception date of a specimen of a patient used for the test.

9. The support method of any one of claims 1 to 8, wherein a default value is set for the set meeting frames.

10. The support method of any one of claims 1 to 9, further comprising receiving change of setting and/or new registration for the scheduled date for holding the expert meeting, wherein
the generating the divergence information comprises generating the divergence information indicating a difference between the predicted meeting frames and the set meeting frames on the scheduled date for which the change of setting and/or the new registration have been performed, such that the difference is recognizable.

11. The support method of claim 10, wherein the receiving the change of setting and/or the new registration for the scheduled date comprises receiving information of at least one selected from a number of meetings and a meeting time.

12. The support method of any one of claims 1 to 8, wherein the set meeting frames are set based on information obtained from a number of previously held expert meetings.

13. The support method of claim 1, further comprising displaying the divergence information.

14. The support method of claim 13, wherein
the generating the divergence information comprises generating the divergence information based on the request information of a plurality of the panel tests on each of a plurality of the scheduled dates for holding the expert meeting, and
the displaying the divergence information comprises displaying a plurality of candidate dates for holding the expert meeting and a plurality of pieces of the generated divergence information so as to associate the plurality of candidate dates and the plurality of pieces of the generated divergence information with each other.

15. The support method of claim 13 or 14, wherein
the displaying the divergence information comprises displaying at least one selected from
a screen including information in which the predicted meeting frames and the set meeting frames on the scheduled date are associated with each other,
a screen including information indicating a difference between the predicted meeting frames and the set meeting frames on the scheduled date, and
a screen including information indicating that the set meeting frames are not sufficient on the scheduled date.

16. The support method of any one of claims 13 to 15, wherein
the request information of each panel test includes information of at least one selected from a medical institution that makes a request, a kind of a disease, and a kind of the test panel, and
the displaying the divergence information comprises displaying the information of the predicted meeting frames in a format based on the information included in the request information.

17. The support method of any one of claims 13 to 16, wherein the displaying the divergence information further comprises displaying link information for displaying a screen for receiving change of setting and/or new registration for the scheduled date for holding the expert meeting.

18. The support method of any one of claims 13 to 17, wherein the displaying the divergence information comprises displaying a screen indicating the divergence information, and the screen for receiving change of setting and/or new registration for the scheduled date for holding the expert meeting, in an aligned manner.

19. The support method of claim 17 or 18, further comprising receiving change of setting and/or new registration for the scheduled date for holding the expert meeting through the screen for receiving change of setting and/or new registration for the scheduled date for holding the expert meeting, wherein
the generating the divergence information comprises generating the divergence information indicating a difference between the predicted meeting frames and the set meeting frames on the scheduled date for which change of setting and/or new registration have been performed.

20. The support method of claim 19, wherein the displaying the divergence information comprises displaying the divergence information on the scheduled date for which change of setting and/or new registration have been performed, such that the divergence information on the scheduled date is distinguished from divergence information on the other scheduled dates.

21. The support method of claim 19 or 20, wherein the generating the divergence information further comprises regenerating the divergence information for the other scheduled date having been set.

22. The support method of any one of claims 1 to 21, further comprising transmitting the divergence information to a terminal, of a user, which is connected to the computer via a network.

23. The support method of claim 22, wherein the divergence information is transmitted when the divergence information satisfies a predetermined condition.

24. The support method of any one of claims 1 to 23, further comprising changing the set meeting frames for the scheduled date.

25. The support method of any one of claims 1 to 24, wherein the set meeting frames for the scheduled date are changed, based on the predicted meeting frames, within time frames that are less than predetermined meeting frames greater than the set meeting frames.

26. The support method of any one of claims 1 to 25, wherein the obtaining the request information of the panel tests comprises obtaining the request information of the panel tests from a plurality of medical institutions.

27. The support method of any one of claims 1 to 25, further comprising receiving reservation for a part of the set meeting frames for the expert meeting on the scheduled date, wherein
the generating the information of the predicted meeting frames comprises generating information of the predicted meeting frames based on the request information of the plurality of panel tests and the reservation for the part of the set meeting frames.

28. A support apparatus for supporting, by using a computer, management of an expert meeting in which a plurality of medical workers interpret a result of a panel test for a patient, the support apparatus comprising
a controller, wherein
the controller
obtains request information of a plurality of panel tests for patients for each of whom the expert meeting is held,
generates information of predicted meeting frames that are related to a number of meetings and/or a meeting time and that are predicted on a scheduled date for holding the expert meeting, based on the obtained request information of the plurality of panel tests, and
generates divergence information indicating a difference between the predicted meeting frames and set meeting frames that are related to a number of meetings and/or a meeting time and that are preset on the scheduled date, such that the difference is recognizable.

29. A computer program for supporting, by using a computer, management of an expert meeting in which a plurality of medical workers interpret gene information of a patient, the computer program causing the computer to perform the steps of:
obtaining request information of a plurality of panel tests for patients for each of whom the expert meeting is held;
generating information of predicted meeting frames that are related to a number of meetings and/or a meeting time and that are predicted on a scheduled date for holding the expert meeting, based on the obtained request information of the plurality of panel tests; and
generating divergence information indicating a difference between the predicted meeting frames and set meeting frames that are related to a number of meetings and/or a meeting time and that are preset on the scheduled date, such that the difference is recognizable.

30. A support system for supporting, by using a computer, management of an expert meeting in which a plurality of medical workers interpret gene information of a patient, the support system comprising:
a support apparatus comprising a controller; and
a computer, wherein
the controller of the support apparatus
obtains request information of a plurality of panel tests for patients for each of whom the expert meeting is held,
generates information of predicted meeting frames that are related to a number of meetings and/or a meeting time and that are predicted on a scheduled date for holding the expert meeting, based on the obtained request information of the plurality of panel tests; and
generates divergence information indicating a difference between the predicted meeting frames and set meeting frames that are related to a number of meetings and/or a meeting time and that are preset on the scheduled date, such that the difference is recognizable, and
the computer obtains and displays the divergence information.
